# EUROPEAN PATENT APPLICATION

(11) **EP 3 715 452 A1**
(43) Date of publication of application: **30.09.2020**
(21) Application number: 18880807.5
(22) Date of filing: 22.11.2018
(51) Int. Cl.: C12N 5/0793, A61K 35/30, A61K 35/545, A61L 27/36, A61L 27/38, A61P 27/02, C12N 5/071, C12N 5/079, C12Q 1/02

(54) **PRODUCTION METHOD FOR CELL MASS INCLUDING NEURAL CELLS/TISSUE AND NON-NEURAL EPITHELIAL TISSUE, AND CELL MASS FROM SAME**

(30) Priority: 24.11.2017 JP 2017226308
(71) Applicant: SUMITOMO CHEMICAL COMPANY, LIMITED, Chuo-ku Tokyo 104-8260 (JP)
(72) Inventor: NAKANO, Tokushige, Osaka-shi Osaka 554-8558 (JP)
(74) Representative: J A Kemp LLP
(86) International application number: PCT/JP2018/043280
(87) International publication number: WO 2019/103125

(57) **Abstract**

The present invention aims to provide a method for efficiently producing, from pluripotent stem cells, a cell mass containing a neural cell or neural tissue, and nonneural epithelial tissue. A method for producing a cell mass containing 1) neural cells or neural tissue and 2) nonneural epithelial tissue, including the following steps (1) and (2):
(1) a first step of suspension-culturing pluripotent stem cells to form a cell aggregate in the presence of a Wnt signal transduction pathway inhibiting substance,
(2) a second step of suspension-culturing the aggregate obtained in the first step in the presence of a BMP signal transduction pathway activating substance, thereby obtaining a cell mass comprising 1) neural cells or neural tissue and 2) nonneural epithelial tissue.

## Description

### [Technical Field]

The present invention relates to a method for producing from a pluripotent stem cell a cell mass containing a neural cell or neural tissue, and nonneural epithelial tissue, and a cell mass therefrom.

### [Background Art]

Non-patent document 1 reports that human cornea organoid was produced by suspension culturing of aggregates prepared from human iPS cells in the presence of a Wnt signal transduction pathway inhibiting substance and a mouse sarcoma-derived basement membrane preparation (Matrigel). However, to avoid contamination with xenogeneic components and undetermined factors, a method for producing a cell mass containing a neural cell or neural tissue, and nonneural epithelial tissue has been demanded, which does not require use of a mouse sarcoma-derived basement membrane preparation.

Non-patent document 2 reports that a three-dimensional crystallin lens was produced by adhesion culturing of human iPS cells on a flat plane. Non-patent document 3 reports that a colony composed of central nervous system, retina, cornea, crystallin lens, and epidermis was two-dimensionally formed by adhesion culturing of human iPS cells on a flat plane. However, a method for efficiently producing a cell mass containing a neural cell or neural tissue, and nonneural epithelial tissue by suspension culturing not requiring a container subjected to a specific treatment and permitting easy scaling up has been desired.

Patent document 1 reports that anterior ocular segment tissue such as cornea, crystalline lens, and the like was formed three-dimensionally by forming a cell aggregate in the absence of a Wnt signal transduction pathway inhibiting substance, reacting 5 nM BMP4 with the obtained aggregate for a long time, and performing suspension culturing in a serum-free medium.

However, since the recombinant protein (BMP4) is expensive, a method for producing a cell mass containing a neural cell or neural tissue, and nonneural epithelial tissue which does not require use of a recombinant protein at a high concentration for a long time has been desired.

### [Document List]

### [Patent document]

patent document 1: WO 2015/020091

### [non-patent documents]

non-patent document 1: Foster et al. Scientific Reports, 7, 2017.
non-patent document 2: Fu et al. Investigative Ophthalmology & Visual Science, 58.1, 2017: 517-527.
non-patent document 3: Hayashi et al. Nature, 531.7594, 2016: 376-380.

### [SUMMARY OF THE INVENTION]

### [Problems to be Solved by the Invention]

An object of the present invention is to provide a method for efficiently producing, from pluripotent stem cells, a cell mass containing a neural cell or neural tissue, and nonneural epithelial tissue. Particularly, it aims to provide a method for efficiently producing a cell mass which uses a feeder-free cultured pluripotent stem cell as a starting material, permits reduction of the amount of expensive recombinant protein to be used, and produces the cell mass at a lower cost.

### [Means of Solving the Problems]

The present inventors have conducted intensive studies in an attempt to solve the aforementioned problems and found that a cell mass containing a neural cell or neural tissue, and nonneural epithelial tissue can be produced efficiently by suspension culturing in the presence of a Wnt signal transduction pathway inhibiting substance and addition of a BMP signal transduction pathway activating substance. Furthermore, they have found that production efficiency of a cell mass can be improved by treating a pluripotent stem cell with 1) a TGFβ family signal transduction pathway inhibiting substance and/or a Sonic hedgehog signal transduction pathway activating substance in the absence of a feeder cell. In addition, they have succeeded in differentiation induction of a cell mass containing a neural cell or neural tissue, and nonneural epithelial tissue by BMP4 at a lower concentration than before by optimizing the addition conditions of the BMP signal transduction pathway activating substance, and identifying the optimal time for the addition to be within 72 hr from the start of suspension culturing.

That is, the present invention relates to the following.
[1] A method for producing a cell mass comprising 1) neural cells or neural tissue and 2) nonneural epithelial tissue, comprising the following steps (1) and (2):
   (1) a first step of suspension-culturing pluripotent stem cells to form a cell aggregate in the presence of a Wnt signal transduction pathway inhibiting substance,
   (2) a second step of suspension-culturing the aggregate obtained in the first step in the presence of a BMP signal transduction pathway activating substance, thereby obtaining a cell mass comprising 1) neural cells or neural tissue and 2) nonneural epithelial tissue.
[2] A method for producing a cell mass comprising 1) neural cells or neural tissue and 2) nonneural epithelial tissue, comprising the following steps (a), (1) and (2):
   (a) step a of maintenance-culturing pluripotent stem cells in the absence of feeder cells and in a medium containing 1) a TGFβ family signal transduction pathway inhibiting substance and/or a Sonic hedgehog signal transduction pathway activating substance, and 2) a factor for maintaining an undifferentiated state,
      (1) a first step of suspension-culturing the pluripotent stem cells, which were maintenance-cultured in step a, to form a cell aggregate in the presence of a Wnt signal transduction pathway inhibiting substance,
      (2) a second step of suspension-culturing the aggregate obtained in the first step in the presence of a BMP signal transduction pathway activating substance, thereby obtaining a cell mass comprising 1) neural cells or neural tissue and 2) nonneural epithelial tissue.
[3] The production method of the above-mentioned [1] or [2], wherein the BMP signal transduction pathway activating substance in the step (2) is added within 0.5 hr to 72 hr from the start of the suspension culturing of the pluripotent stem cells in the step (1).
[4] The production method of the above-mentioned [1] or [2], wherein the BMP signal transduction pathway activating substance in the step (2) is added during a period when not less than 10% of the cells of the surface layer of the aggregate formed in the step (1) form a tight junction.
[5] The production method of any of the above-mentioned [1] to [4], wherein the BMP signal transduction pathway activating substance is at least one kind of protein selected from the group consisting of BMP2, BMP4, BMP7, BMP13, and GDF7.
[6] The production method of any of the above-mentioned [1] to [4], wherein the BMP signal transduction pathway activating substance is BMP4.
[7] The production method of the above-mentioned [6], wherein the suspension culturing in the step (2) is performed in a medium with a concentration of the BMP4 of 10 pM - 5 nM.
[8] The production method of any of the above-mentioned [1] to [7], wherein the Wnt signal transduction pathway inhibiting substance has an inhibitory activity against non-canonical Wnt pathway.
[9] The production method of any of the above-mentioned [1] to [8], wherein the Wnt signal transduction pathway inhibiting substance is a PORCN inhibitor.
[10] The production method of the above-mentioned [9], wherein the PORCN inhibitor is at least one kind of compound selected from the group consisting of IWP-2, IWP-3, IWP-4, IWP-L6, IWP-12, LGK-974, Wnt-C59, ETC-159, and GNF-6231.
[11] The production method of any of the above-mentioned [1] to [7], wherein the Wnt signal transduction pathway inhibiting substance is a TANK inhibitor.
[12] The production method of the above-mentioned [11], wherein the TANK inhibitor is at least one kind of compound selected from the group consisting of IWR1-endo, XAV939, and MN-64.
[13] The production method of any of the above-mentioned [1] to [12], wherein the culturing in the step (1) and/or the step (2) is performed in the further presence of a TGFβ signal transduction pathway inhibiting substance.
[14] The production method of the above-mentioned [13], wherein the TGFβ signal transduction pathway inhibiting substance is an Alk5/TGFβR1 inhibitor.
[15] The production method of the above-mentioned [14], wherein the Alk5/TGFβR1 inhibitor is at least one kind of compound selected from the group consisting of SB431542, SB505124, SB525334, LY2157299, GW788388, LY364947, SD-208, EW-7197, A 83-01, and RepSox.
[16] The production method of any of the above-mentioned [2] to [15], wherein the Sonic hedgehog signal transduction pathway activating substance in the step (a) is at least one kind of compound selected from the group consisting of SAG, Purmorphamine, and GSA-10.
[17] The production method of the above-mentioned [16], wherein the concentration of the Sonic hedgehog signal transduction pathway activating substance contained in the medium in the step (a) is a concentration showing a Sonic hedgehog signal transduction promoting activity corresponding to that of 10 nM to 700 nM SAG.
[18] The production method of any of the above-mentioned [1] to [17], wherein the suspension culturing in the step (1) and/or step (2) is suspension culturing using a serum-free medium.
[19] The production method of the above-mentioned [18], wherein the serum-free medium is a serum-free medium containing a serum replacement.
[20] A cell mass comprising 1) neural cells or neural tissue and 2) nonneural epithelial tissue obtained by the production method of any of the above-mentioned [1] to [19].
[21] A method for producing a nonneural epithelial tissue sheet, comprising the following steps (1) - (4):
   (1) a first step of suspension-culturing pluripotent stem cells to form a cell aggregate in the presence of a Wnt signal transduction pathway inhibiting substance,
   (2) a second step of suspension-culturing the aggregate obtained in the first step in the presence of a BMP signal transduction pathway activating substance, thereby obtaining a cell mass comprising 1) neural cells or neural tissue and 2) nonneural epithelial tissue,
   (3) a third step of collecting 2) nonneural epithelial tissue from the cell mass obtained in the second step,
   (4) a fourth step of dispersing the 2) nonneural epithelial tissue obtained in the third step and culturing same on a flat plane, thereby obtaining a nonneural epithelial tissue sheet.
[22] A method for producing a nonneural epithelial tissue sheet, comprising the following step (a) and the following steps (1) - (4) :
   (a) step a of maintenance-culturing pluripotent stem cells in the absence of feeder cells and in a medium containing 1) a TGFβ family signal transduction pathway inhibiting substance and/or a Sonic hedgehog signal transduction pathway activating substance, and 2) a factor for maintaining an undifferentiated state,
      (1) a first step of suspension-culturing the pluripotent stem cells, which were maintenance-cultured in step a, to form a cell aggregate in the presence of a Wnt signal transduction pathway inhibiting substance,
      (2) a second step of suspension-culturing the aggregate obtained in the first step in the presence of a BMP signal transduction pathway activating substance, thereby obtaining a cell mass comprising 1) neural cells or neural tissue and 2) nonneural epithelial tissue,
      (3) a third step of collecting 2) nonneural epithelial tissue from the cell mass obtained in the second step,
      (4) a fourth step of dispersing the 2) nonneural epithelial tissue obtained in the third step and culturing same on a flat plane, thereby obtaining a nonneural epithelial tissue sheet.
[23] The production method of the above-mentioned [21] or [22], wherein the method for collecting the nonneural epithelial tissue in the step (3) is performed by freeze-thawing of the cell mass.
[24] The production method of the above-mentioned [23], wherein the nonneural epithelial tissue is collected by freeze-thawing the cell mass according to a slow freezing method.
[25] The production method of any of the above-mentioned [21] to [24], wherein the nonneural epithelial tissue of the 2) is cornea or a precursor tissue thereof.
[26] A nonneural epithelial tissue sheet obtained by the production method of any of the above-mentioned [21] to [25].
[27] A cell mass comprising 1) neural cells or neural tissue and 2) nonneural epithelial tissue, wherein not less than 30% of the surface of the 1) neural cells or neural tissue is coated with 2) nonneural epithelial tissue, and wherein
   a space having a distance between the 1) neural cells or neural tissue and the 2) nonneural epithelial tissue on the outer side of not less than 30 µm is formed in at least a part of the surface region of the 1) neural cells or neural tissue coated with the 2) nonneural epithelial tissue.
[28] The cell mass of the above-mentioned [27], wherein the 2) nonneural epithelial tissue is a nonneural epithelial tissue capable of maintaining a sphere-like structure autonomously formed by epithelial cells even in a culture medium.
[29] The cell mass of the above-mentioned [27] or [28], wherein the 2) nonneural epithelial tissue has epithelial cell polarity.
[30] The cell mass of any of the above-mentioned [27] to [29], wherein the 2) nonneural epithelial tissue has a basement membrane-like structure.
[31] The cell mass of the above-mentioned [30], wherein the basement membrane-like structure is formed between the 1) neural cells or neural tissue and the 2) nonneural epithelial tissue.
[32] The cell mass of any of the above-mentioned [27] to [31], wherein the 2) nonneural epithelial tissue is pseudostratified epithelium.
[33] The cell mass of any of the above-mentioned [27] to [31], wherein the 2) nonneural epithelial tissue is stratified epithelium.
[34] The cell mass of any of the above-mentioned [27] to [33], wherein the 2) nonneural epithelial tissue is cornea or precursor tissue thereof.
[35] The cell mass of any of the above-mentioned [27] to [34], wherein the 1) neural cells or neural tissue are/is central nervous system cells or tissue or precursor tissue thereof.
[36] The cell mass of the above-mentioned [35], wherein the central nervous system cell or tissue is retina.
[37] The cell mass of any of the above-mentioned [27] to [36], wherein a part of the 2) nonneural epithelial tissue is placode or placode-derived tissue.
[38] The cell mass of the above-mentioned [37], wherein the placode is cranial placode.
[39] The cell mass of the above-mentioned [37], wherein the placode-derived tissue is crystalline lens.
[40] A method for evaluating toxicity or efficacy of a test substance, comprising
   a step of bringing the cell mass comprising 1) neural cells or neural tissue and 2) nonneural epithelial tissue of any of the above-mentioned [27] to [39] into contact with a test substance, and
   a step of detecting an influence of the test substance on the cells or tissue.
[41] A method for evaluating toxicity or efficacy of a test substance, comprising a step of bringing a cell mass comprising 1) neural cells or neural tissue and 2) nonneural epithelial tissue obtained by the production method of any of the above-mentioned [1] to [19] into contact with a test substance, and
   a step of detecting an influence of the test substance on the cells or tissue.
[42] A method for evaluating toxicity or efficacy of a test substance, comprising a step of bringing a nonneural epithelial tissue sheet obtained by the method of any of the above-mentioned [21] to [25] into contact with a test substance, and
   a step of detecting an influence of the test substance on the nonneural epithelial tissue sheet.
[43] The method of any of the above-mentioned [40] to [42], wherein the detection step includes
   a step of staining with a dye the cell mass or nonneural epithelial tissue sheet after contact with the test substance,
   a step of extracting the dye from the stained cell mass or nonneural epithelial tissue sheet, and
   a step of quantifying the amount of the extracted dye to evaluate stimulability of the test substance.
[44] A therapeutic drug for a disease based on a disorder of a sensory organ, comprising a cell mass obtained by the method of any of the above-mentioned [1] to [19].
[45] A therapeutic drug for a disease based on a disorder of cornea, comprising a nonneural epithelial tissue sheet obtained by the method of the above-mentioned [25].
[46] A method for treating a disease based on a disorder of a sensory organ in a non-human animal, comprising a step of transplanting an effective amount of 2) nonneural epithelial tissue from a cell mass obtained by the method of any of the above-mentioned [1] to [19] to a target in need of the transplantation.
[47] A reagent for evaluating toxicity or efficacy of a test substance, comprising a cell mass obtained by the method of any of the above-mentioned [1] to [19].

### [Effect of the Invention]

According to the present invention, a cell mass containing a neural cell or neural tissue and nonneural epithelial tissue can be produced efficiently from pluripotent stem cells at a low cost.

In addition, a cell mass with a space between the neural cells or neural tissue and the nonneural epithelial tissue, that can easily separate and collect the nonneural epithelial tissue from the neural cells or neural tissue can be provided.

### [Brief Description of the Drawings]

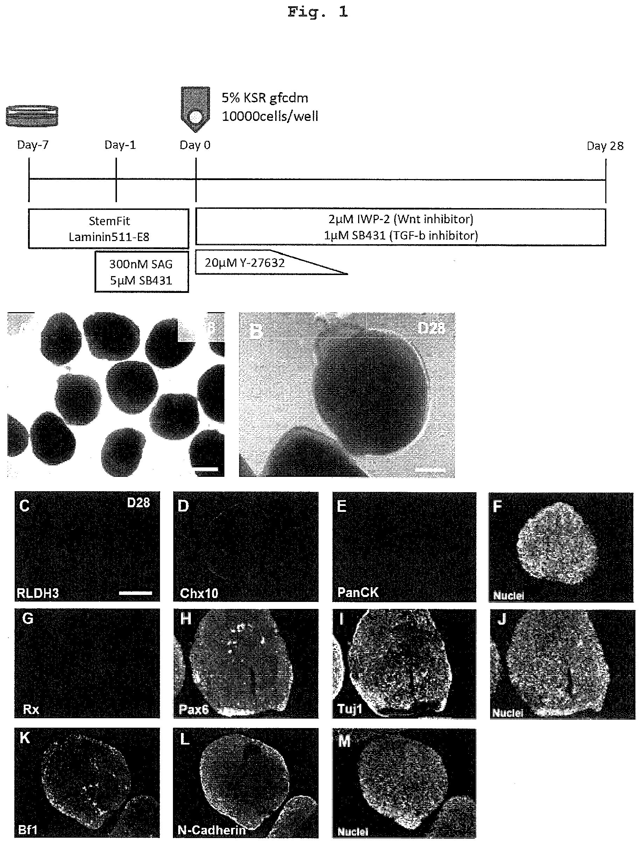

[Fig. 1] The upper panel of Fig. 1 is a diagram schematically showing a procedure for producing a cell mass from human ES cells in Comparative Example 1. The lower panels A and B are diagrams showing bright-field observation images by an inverted microscope of the cell mass 28 days after the start of suspension culturing in Comparative Example 1. The lower panels C - M show the results of examination by fluorescent immunostaining of the expression state of each cell marker in the cell mass 28 days after the start of suspension culturing. C - F respectively show RLDH3, Chx10, pan-cytokeratin (Pan CK) and nuclear-stained image thereof. G - J respectively show Rx, Pax6, βIII tubulin (Tuj1) and nuclear-stained image thereof. K - M respectively show Bf1, N-Cadherin and nuclear-stained image thereof.

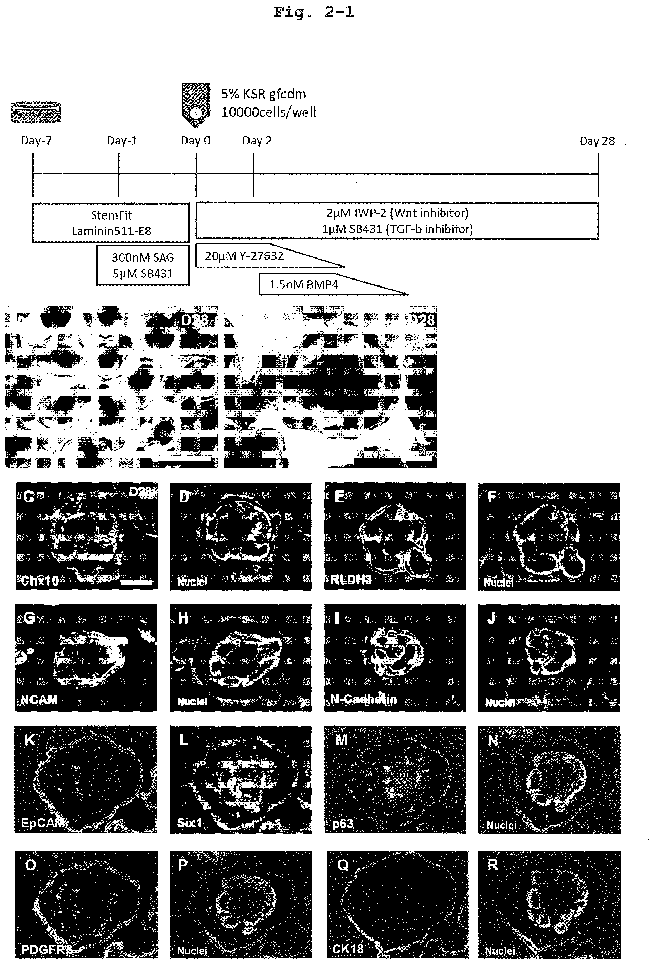

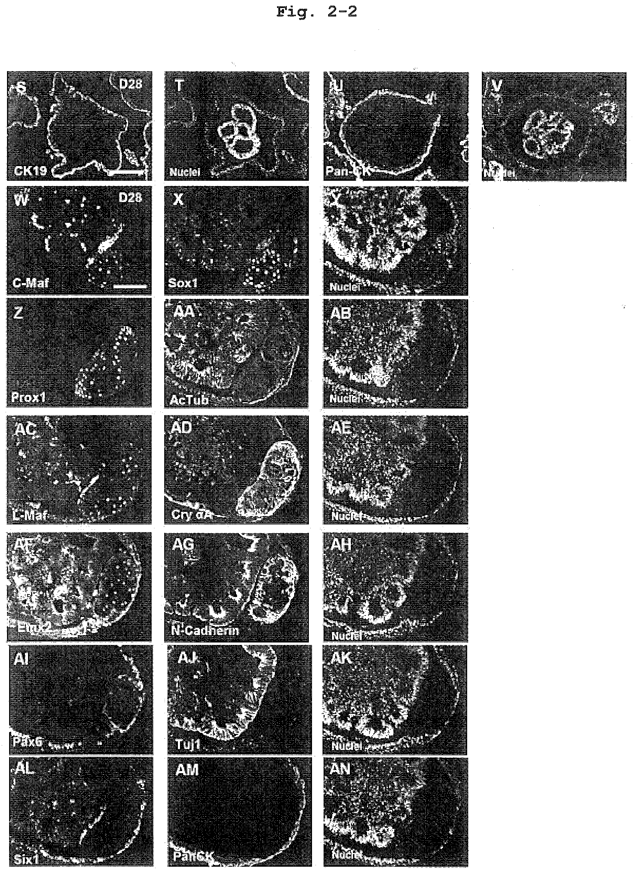

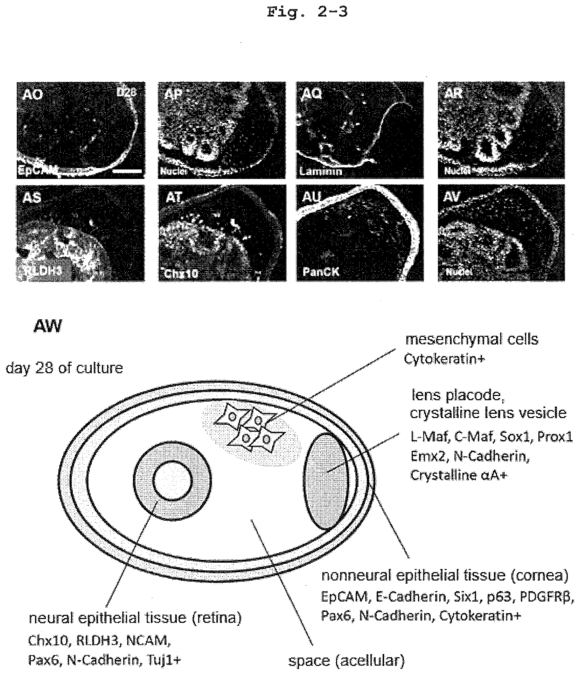

[Fig. 2-1] The upper panel of Fig. 2-1 is a diagram schematically showing a procedure for producing a cell mass containing neural tissue and nonneural epithelial tissue from human ES cells in Example 1. The lower panels A and B are diagrams showing bright-field observation images by an inverted microscope of the cell mass 28 days after the start of suspension culturing in Example 2. The lower panels C - R show the results of examination by fluorescent immunostaining of the expression state of each cell marker in the cell mass 28 days after the start of suspension culturing. C and D respectively show stained image of Chx10 and nuclear-stained image thereof. E and F respectively show stained image of RLDH3 and nuclear-stained image thereof. G and H respectively show stained image of NCAM and nuclear-stained image thereof. I and J respectively show stained image of N-Cadherin and nuclear-stained image thereof. K - N respectively show stained images of EpCAM, Six1, p63 and nuclear-stained image thereof. O and P respectively show stained image of PDGFRβ and nuclear-stained image thereof. Q and R respectively show stained image of cytokeratin 18 (CK18) and nuclear-stained image thereof.
[Fig. 2-2] S - AN of Fig. 2-2 show the results of examination by fluorescent immunostaining of the expression state of each cell marker in the cell mass 28 days after the start of suspension culturing. S and T respectively show stained image of cytokeratin 19 (CK19) and nuclear-stained image thereof. U and V respectively show stained image of pan-cytokeratin and nuclear-stained image thereof. W - Y respectively show stained images of C-Maf, Sox1 and nuclear-stained image thereof. Z - AB respectively show stained images of Prox1, acetylated tubulin (AcTub) and nuclear-stained image thereof. AC - AE respectively show stained images of L-Maf, Crystalline αA (Cry αA) and nuclear-stained image thereof. AF - AH respectively show stained images of Emx2, N-Cadherin and nuclear-stained image thereof. AI - AK respectively show stained images of Pax6, βIII tubulin and nuclear-stained image thereof. AL - AN respectively show stained images of Six1, pan-cytokeratin and nuclear-stained image thereof.
[Fig. 2-3] AO - AV of Fig. 2-3 show the results of examination by fluorescent immunostaining of the expression state of each cell marker in the cell mass 28 days after the start of suspension culturing. AO - AP respectively show stained image of EpCAM and nuclear-stained image thereof. AQ - AR respectively show stained image of Laminin and nuclear-stained image thereof. AS - AV respectively show stained images of RLDH3, Chx10, pan-cytokeratin and nuclear-stained image thereof. The lower panel AW is a diagram schematically showing the structure of a cell mass containing neural tissue and nonneural epithelial tissue on day 28 of culturing.

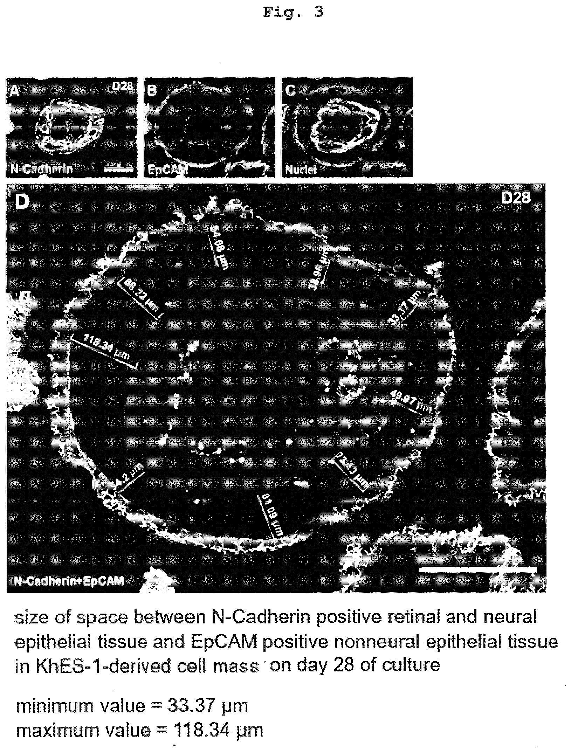

[Fig. 3] Fig. 3A - C show the results of examination by fluorescent immunostaining of the expression state of each cell marker in the cell mass 28 days after the start of suspension culturing in Example 2. A - C respectively show stained images of N-Cadherin and EpCAM and nuclear-stained image thereof. The lower panel of Fig. 3D shows the results of measurement, using analysis software, of images obtained by fluorescence immunostaining.

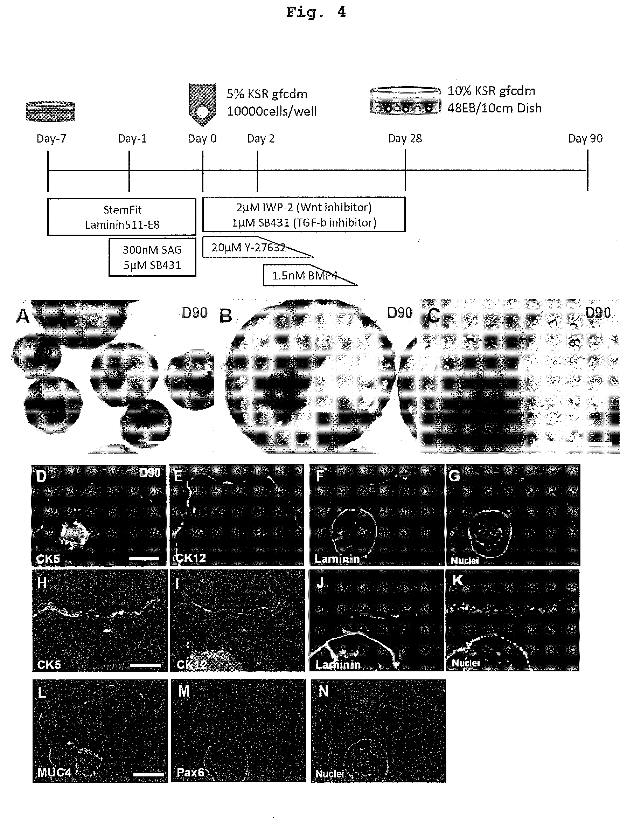

[Fig. 4] The upper panel of Fig. 4 is a diagram schematically showing a procedure for producing a cell mass containing neural tissue and nonneural epithelial tissue from human ES cells in Example 3. The lower panels A - C are diagrams showing bright-field observation images by an inverted microscope of the cell mass 90 days after the start of suspension culturing in Example 3. The lower panels D - N show the results of examination by fluorescent immunostaining of the expression state of each cell marker in the cell mass 90 days after the start of suspension culturing. D - K respectively show stained images of cytokeratin 5 (CK5), cytokeratin 12 (CK12) and Laminin and nuclear-stained image thereof. L - N respectively show stained images of Mucin4 (MUC4) and Pax6 and nuclear-stained image thereof.

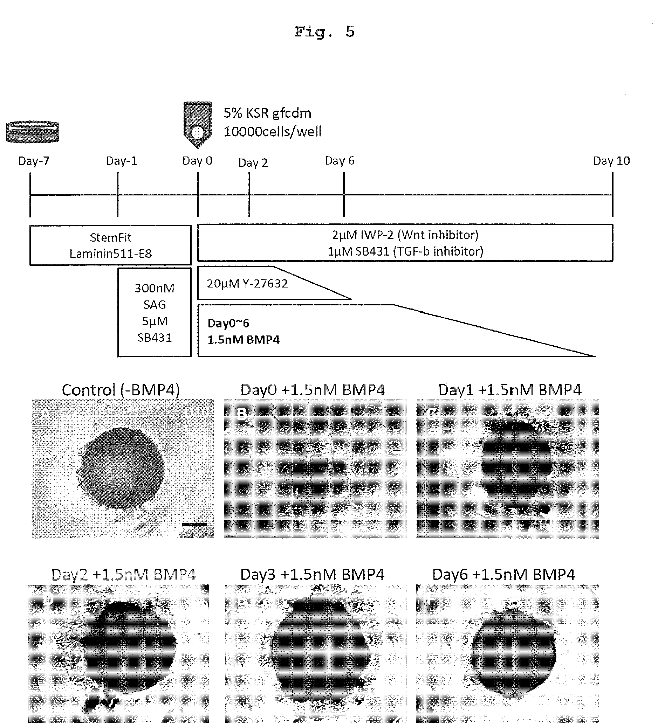

[Fig. 5] The upper panel of Fig. 5 is a diagram schematically showing a procedure for producing a cell mass containing neural tissue and nonneural epithelial tissue from human ES cells by changing the time of addition of BMP4 in Example 4. The lower panels A - F are diagrams showing bright-field observation images by an inverted microscope of the cell masses 10 days after the start of suspension culturing in Example 2. Diagrams of bright-field observation images by an inverted microscope of the cell masses 10 days after the start of suspension culturing and formed from A: control cells without addition of BMP4, B: cells added with BMP4 simultaneously with the start of suspension culturing, and C - F: cells added with BMP4 on days 1, 2, 3 and 6, respectively, after the start of suspension culturing.

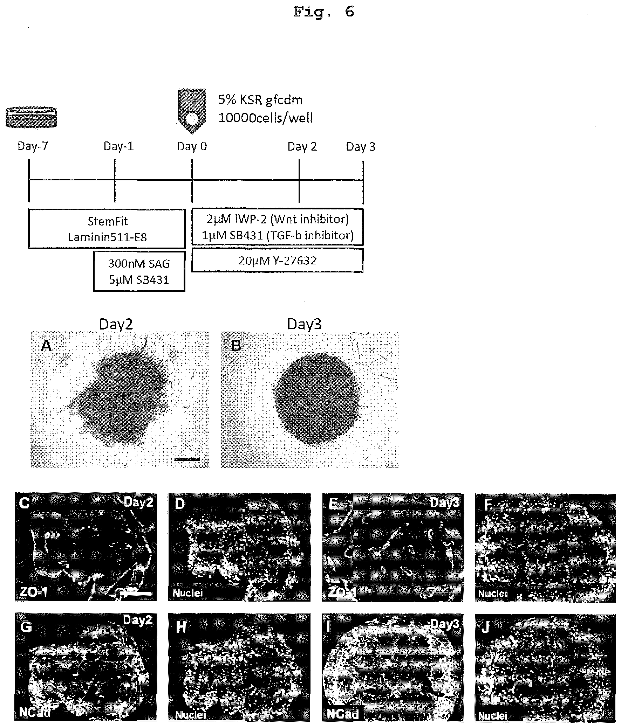

[Fig. 6] The upper panel of Fig. 6 is a diagram schematically showing a procedure for preparing a cell aggregate in the production process of a cell mass containing neural tissue and nonneural epithelial tissue from human ES cells in Example 5. The lower panels A and B are diagrams showing bright-field observation images by an inverted microscope of the cell aggregate respectively 2 and 3 days after the start of suspension culturing in Example 5. The lower panels C - J show the results of examination by fluorescent immunostaining of the expression state of each cell marker in the cell aggregate 2 or 3 days after the start of suspension culturing. C and D respectively show stained images of ZO-1 and nuclear-stained image thereof in the cell aggregate 2 days after the start of suspension culturing. G and H respectively show stained images of N-Cadherin (NCad) and nuclear-stained image thereof in the cell aggregate 2 days after the start of suspension culturing. E and F respectively show stained images of ZO-1 and nuclear-stained image thereof in the cell aggregate 3 days after the start of suspension culturing. I and J respectively show stained images of N-Cadherin (NCad) and nuclear-stained image thereof in the cell aggregate 3 days after the start of suspension culturing.

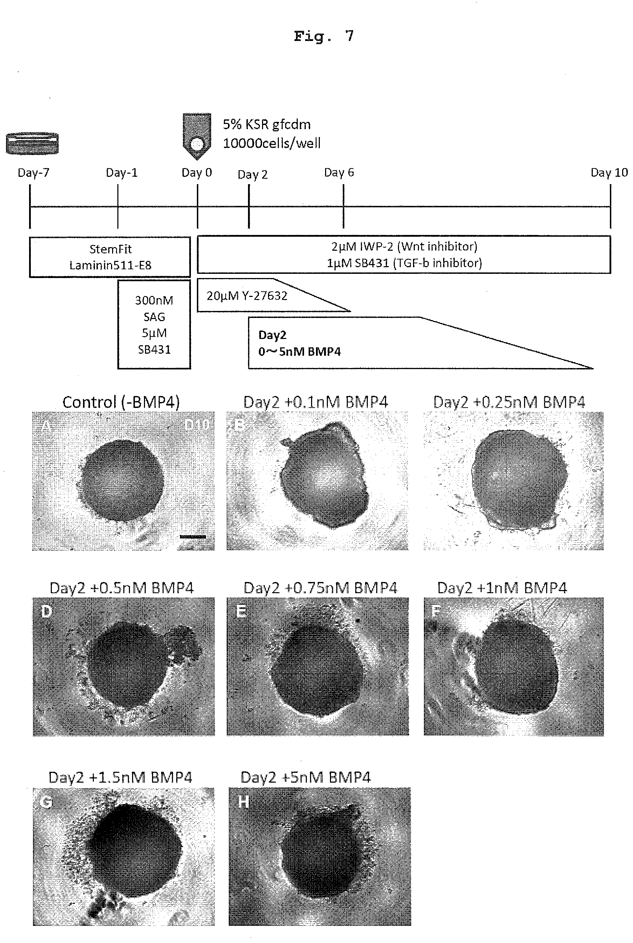

[Fig. 7] The upper panel of Fig. 7 is a diagram schematically showing a procedure for producing a cell mass containing neural tissue and nonneural epithelial tissue from human ES cells by changing the concentration of BMP4 added in Example 6. The lower panels A - H are diagrams showing bright-field observation images by an inverted microscope of the cell masses 10 days after the start of suspension culturing in Example 6. Diagrams of bright-field observation images by an inverted microscope of the cell masses 10 days after the start of suspension culturing and formed from A: control cells without addition of BMP4, and B - H: cells obtained by adding BMP4 at varied concentrations (0.1 nM, 0.25 nM, 0.5 nM, 0.75 nM, 1 nM, 1.5 nM, 5 nM) on day 2 after the start of suspension culturing and further performing suspension culturing.

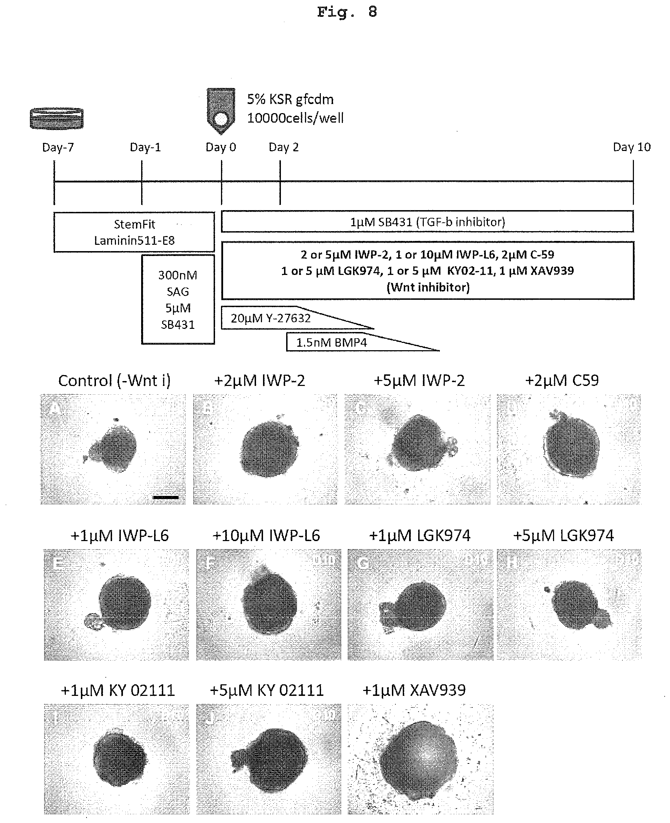

[Fig. 8] The upper panel of Fig. 8 is a diagram schematically showing a procedure for examining the effect of each Wnt signal transduction pathway inhibiting substance on the production of a cell mass containing neural tissue and nonneural epithelial tissue from human ES cells in Example 7. The lower panels A - K are diagrams showing bright-field observation images by an inverted microscope of the cell mass 28 days after the start of suspension culturing in Example 7. Diagrams of bright-field observation images by an inverted microscope of the cell masses 10 days after the start of suspension culturing and formed from A: control without addition of a Wnt signal transduction pathway inhibiting substance, and B - K: cells obtained by adding various kinds of Wnt signal transduction pathway inhibiting substance at varied concentrations at the start of suspension culturing and further performing suspension culturing.

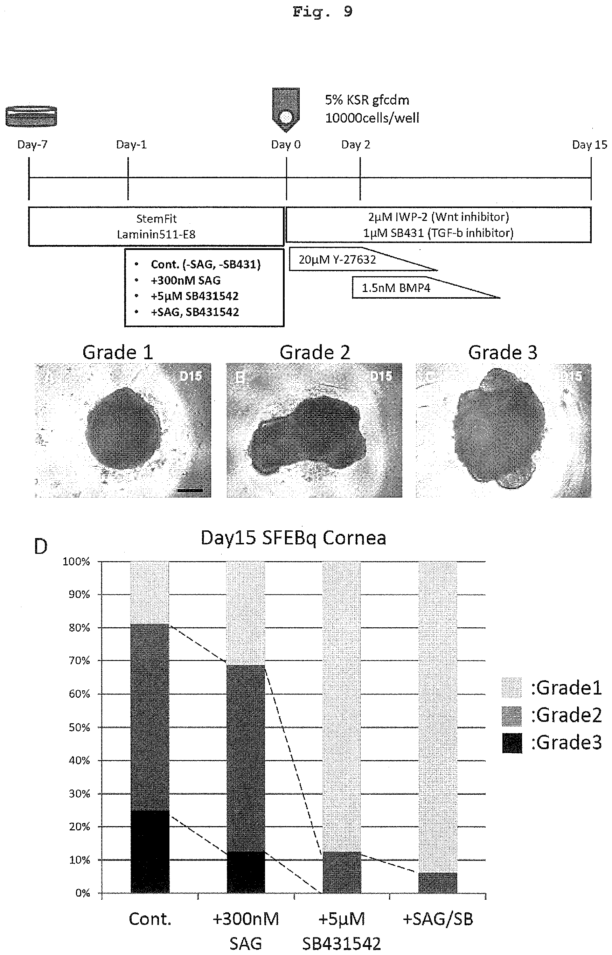

[Fig. 9] The upper panel of Fig. 9 is a diagram schematically showing a procedure for examining the effect of a pre-treatment with a compound before the start of suspension culturing on the production of a cell mass containing neural tissue and nonneural epithelial tissue from human ES cells in Example 8. The lower panels A - C are diagrams showing bright-field observation images by an inverted microscope of the cell mass 15 days after the start of suspension culturing in Example 8. A is an example of a nearly spherical Grade 1 cell mass in which not less than 80% of the entire circumference is covered with nonneural epithelium, B is an example of a Grade 2 cell mass in which 80% to 40% of the entire circumference is covered with nonneural epithelium or which is irregularly shaped; and C is an example of a Grade 3 cell mass in which a ratio of nonneural epithelium on the surface of the cell mass is not more than 40%. D is a graph showing the results of quality evaluation of the cell masses formed after a compound pre-treatment under respective conditions.

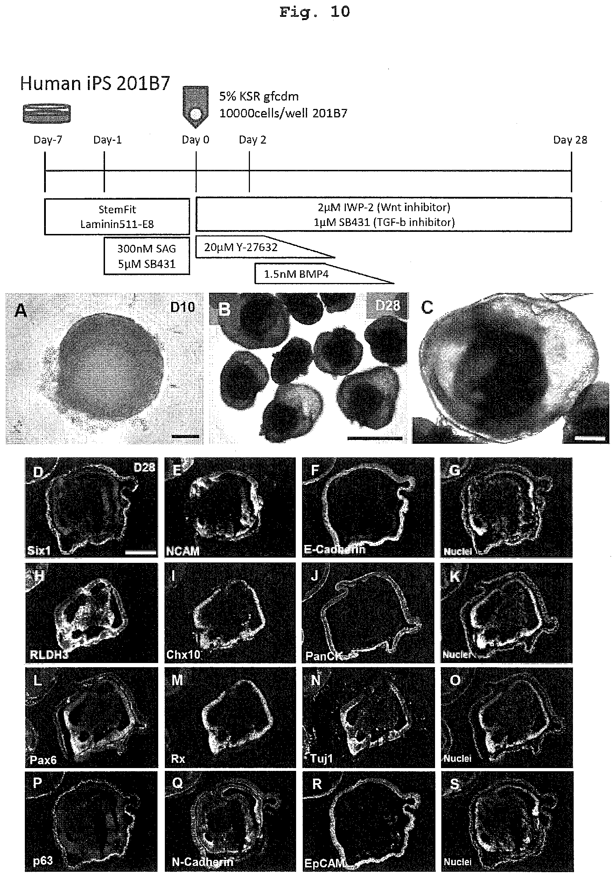

[Fig. 10] The upper panel of Fig. 10 is a diagram schematically showing a procedure for producing a cell mass containing neural tissue and nonneural epithelial tissue from human ES cells in Example 9. The lower panel A is a diagram showing bright-field observation image by an inverted microscope of the cell mass 10 days after the start of suspension culturing in Example 9. B and C are diagrams showing bright-field observation images by an inverted microscope of the cell masses 28 days after the start of suspension culturing in Example 9. The lower panels D - S show the results of examination by fluorescent immunostaining of the expression state of each cell marker in the cell mass 28 days after the start of suspension culturing. D - G respectively show stained images of Six1, NCAM and E-Cadherin and nuclear-stained image thereof. H - K respectively show stained images of RLDH3, Chx10 and pan-cytokeratin and nuclear-stained image thereof. L - O respectively show stained images of Pax6, Rx and βIII tubulin and nuclear-stained image thereof. P - S respectively show stained images of p63, N-Cadherin and EpCAM and nuclear-stained image thereof.

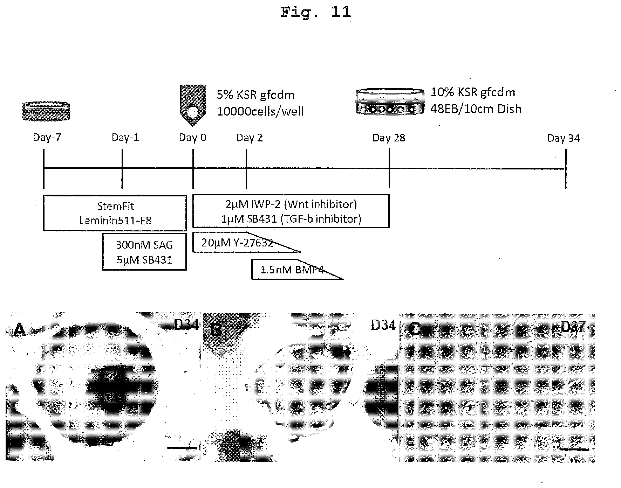

[Fig. 11] The upper panel of Fig. 11 is a diagram schematically showing a procedure for producing a cell mass containing neural tissue and nonneural epithelial tissue from human ES cells in Example 10. The lower panels are diagrams showing bright-field observation images by an inverted microscope of the cell masses in Example 10, in which A is a cell mass 34 days after the start of suspension culturing, B is nonneural epithelial tissue alone isolated from A, and C is isolated nonneural epithelial tissue converted to single cells by an enzyme treatment and on day 3 after being seeded in a cell culture dish.

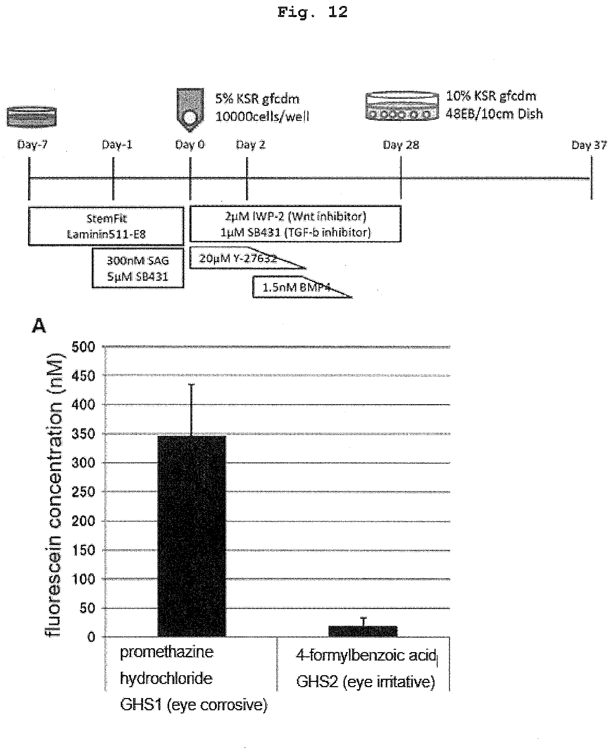

[Fig. 12] The upper panel of Fig. 12 is a diagram schematically showing a procedure for producing a cell mass containing neural tissue and nonneural epithelial tissue from human ES cells in Example 11. The lower panel A is a graph showing the results of evaluation of the cell masses by a fluorescein staining method after treating the cell masses with compounds of GHS classifications 1 and 2 in Example 11.

### [Description of Embodiments]

### 1. Definition

In the present invention, "stem cell" means an undifferentiated cell having differentiation potency and proliferative capacity (particularly self-renewal competence) maintaining differentiation potency. The stem cell includes subpopulations such as pluripotent stem cell, multipotent stem cell, unipotent stem cell and the like according to the differentiation potency. Pluripotent stem cell refers to a stem cell capable of being cultured in vitro and having a potency to differentiate into any cell constituting living organisms (tissue derived from three germ layers (ectoderm, mesoderm, endoderm) (pluripotency). The multipotent stem cell means a stem cell having a potency to differentiate into plural types of tissues or cells, though not all kinds. The unipotent stem cell means a stem cell having a potency to differentiate into a particular tissue or cell.

Pluripotent stem cell can be induced from fertilized egg, clone embryo, germ stem cell, stem cell in a tissue, somatic cell or the like. Examples of the pluripotent stem cell include embryonic stem cell (ES cell), EG cell (embryonic germ cell), and induced pluripotent stem cell (iPS cell). Muse cell (Multi-lineage differentiating stress enduring cell) obtained from mesenchymal stem cell (MSC), and GS cell produced from reproductive cell (e.g., testis) are also encompassed in the pluripotent stem cell. Embryonic stem cell was first established in 1981, and has also been applied to the generation of knockout mouse since 1989. In 1998, human embryonic stem cell was established, which is also being utilized for regenerative medicine. ES cell can be produced by culturing an inner cell mass on a feeder cell or in a medium containing LIF. The production methods of ES cell are described in, for example, WO 96/22362, WO 02/101057, US 5,843,780, US 6,200,806, and US 6,280,718. Embryonic stem cells are available from given organizations, or a commercially available product can be purchased. For example, human embryonic stem cells, KhES-1, KhES-2 and KhES-3, are available from Kyoto University's Institute for Frontier Medical Sciences. EB5 cell, which is a mouse embryonic stem cell, is available from Incorporated Administrative Agency RIKEN, and D3 cell line, which is a mouse embryonic stem cell, is available from ATCC. Nuclear transfer ES cell (ntES cell), which is one of the ES cells, can be established from a clone embryo produced by transplanting the cell nucleus of a somatic cell into an enucleated egg.

EG cell can be produced by culturing a primordial germ cell in a medium containing mSCF, LIF and bFGF (Cell, 70: 841-847, 1992).

The "induced pluripotent stem cell" in the present invention is a cell induced to have pluripotency by reprogramming a somatic cell by a known method and the like. Specifically, a cell induced to have pluripotency by reprogramming differentiated somatic cells such as fibroblast, and peripheral blood mononuclear cell by the expression of a combination of a plurality of genes selected from the group consisting of reprogramming genes including Oct3/4, Sox2, Klf4, Myc (c-Myc, N-Myc, L-Myc), Glis1, Nanog, Sall4, lin28, and Esrrb can be mentioned. Induced pluripotent stem cell was established by Yamanaka et al. in mouse cell in 2006 (Cell, 2006, 126(4), pp.663-676). In 2007, Induced pluripotent stem cell was also established from human fibroblast, and has pluripotency and self-renewal competence similar to those of embryonic stem cells (Cell, 2007, 131(5), pp.861-872; Science, 2007, 318(5858), pp.1917-1920; Nat. Biotechnol., 2008, 26(1), pp.101-106). Besides the production method based on direct reprogramming by gene expression, induced pluripotent stem cell can also be obtained from somatic cell by the addition of a compound and the like (Science, 2013, 341, pp. 651-654).

While the somatic cell used for producing induced pluripotent stem cell is not particularly limited, tissue-derived fibroblast, blood-lineage cells (e.g., peripheral blood mononuclear cell, T cell), hepatocyte, pancreatic cell, intestinal epithelial cell, and smooth muscle cell can be mentioned.

When induced pluripotent stem cell is produced by reprogramming by the expression of several kinds of genes (e.g., 4 factors of Oct3/4, Sox2, Klf4, and Myc), the means for gene expression is not particularly limited. Examples of the aforementioned means include an infection method using a virus vector (e.g., retrovirus vector, lentivirus vector, Sendaivirus vector, adenovirus vector, adeno-associated virus vector), a gene transfer method using a plasmid vector (e.g., plasmid vector, episomal vector) (e.g., calcium phosphate method, lipofection method, retronectin method, electroporation method), a gene transfer method using an RNA vector (e.g., calcium phosphate method, lipofection method, electroporation method), and a method with direct injection of protein.

The pluripotent stem cell to be used in the present invention is preferably ES cell or induced pluripotent stem cell.

As the multipotent stem cell, tissue stem cells (also called stem cell in a tissue, tissue-specific stem cell or somatic stem cell) such as hematopoietic stem cell, neural stem cell, retinal stem cell, and mesenchymal stem cell can be mentioned.

Genetically-modified pluripotent stem cells can be produced by using, for example, a homologous recombination technique. Examples of the gene on the chromosome to be modified include a cell marker gene, a histocompatibility antigen gene, a gene related to a disease due to a disorder of neural cell and so on. A target gene on the chromosome can be modified using the methods described in Manipulating the Mouse Embryo, A Laboratory Manual, Second Edition, Cold Spring Harbor Laboratory Press (1994); Gene Targeting, A Practical Approach, IRL Press at Oxford University Press (1993); Biomanual Series 8, Gene Targeting, Making of Mutant Mouse using ES cell, YODOSHA CO., LTD. (1995); and so on.

To be specific, for example, the genome gene of the target gene to be modified (e.g., cell marker gene, histocompatibility antigen gene, disease-related gene and so on) is isolated, and a targetting vector used for homologous recombination of the target gene is produced using the isolated genome gene. The produced targetting vector is introduced into stem cells and the cells that showed homologous recombination between the target gene and the targetting vector are selected, whereby stem cells having the modified gene on the chromosome can be produced.

Examples of the method for isolating genome gene of the target gene include known methods described in Molecular Cloning, A Laboratory Manual, Second Edition, Cold Spring Harbor Laboratory Press (1989), Current Protocols in Molecular Biology, John Wiley & Sons (1987-1997) and so on. The genome gene of the target gene can also be isolated using genomic DNA library screening system (manufactured by Genome Systems), Universal GenomeWalker Kits (manufactured by CLONTECH) and so on.

Production of targetting vector used for homologous recombination of the target gene, and efficient selection of a homologous recombinant can be performed according to the methods described in Gene Targeting, A Practical Approach, IRL Press at Oxford University Press (1993); Biomanual Series 8, Gene Targeting, Making of Mutant Mouse using ES cell, YODOSHA CO., LTD. (1995); and so on. As the targetting vector, any of replacement type or insertion type can be used. As the selection method, methods such as positive selection, promoter selection, negative selection, polyA selection and so on can be used.

Examples of a method for selecting the desired homologous recombinant from the selected cell lines include Southern hybridization method, PCR method and so on for the genomic DNA.

The "mammal" in the present invention encompasses rodents, ungulata, carnivora, and primates. The rodents encompass mouse, rat, hamster, and guinea pig. Ungulata encompass swine, bovine, goat, horse, and sheep. Carnivora encompasses dog, and cat. The "primates" in the present invention refers to mammals belonging to the primate, and the primates include prosimian such as lemur, loris, tupai etc, and anthropoidea such as monkey, ape, and human.

The pluripotent stem cells to be used in the present invention are mammalian pluripotent stem cells, preferably pluripotent stem cells of rodents (e.g., mouse, rat) or primates (e.g., human, monkey), most preferably a human pluripotent stem cell.

The "signal transduction" in the present invention refers to the mechanism of the cells for transmission of information, amplification and processing of, and response to biochemical stimulation, such as processes and mechanisms in which receptor proteins present in the cell membrane and the like bind to chemical substances and the like to cause a structural change, which in turn is transmitted sequentially in the cell as a stimulation to finally cause reactions such as gene expression, channel opening and the like.

The "cell adhesion" in the present invention refers to cell-cell adhesion and cell-extracellular matrix adhesion. Adhesion of cells to culture vessels and the like that occurs under an artificial culture environment in vitro is also included in the cell adhesion. As the kind of the cell adhesion, anchoring junction, communicating junction, occluding junction can be mentioned.

The "Tight junction" in the present invention refers to, among cell-cell adhesions, occluding junctions found in vertebrates and chordates. A tight junction is formed between epithelial cells. Whether a tight junction is present in tissues of biological origin or cell masses produced by the production method of the present invention and the like can be detected by, for example, methods such as immunohistochemistry and the like using an antibody (anti-claudin antibody, and anti-ZO-1 antibody) to a constituent component of the tight junction.

The "suspension culturing" or "suspension culturing method" in the present invention refers to culturing while maintaining a state in which cells, cell aggregates or cell masses are suspended in a culture medium and a method of performing the culturing. That is, the suspension culturing is performed under conditions in which cells, cell aggregates or cell masses are not adhered to a culture vessel and the like, and culturing performed under conditions permitting adhesion to a culture vessel and the like (adhesion culturing or adhesion culturing method) is not included in the category of suspension culturing. In this case, adhesion of cell means that a strong cell-substratum junction, which is one type of cell adhesion, is formed between a cell, cell aggregate or cell mass and a culture vessel. More particularly, suspension culturing refers to culturing under conditions in which a strong cell-substratum junction is not formed between a cell, cell aggregate or cell mass and a culture vessel, and adhesion culturing refers to culturing under conditions in which a strong cell-substratum junction is formed between a cell, cell aggregate or cell mass and a culture vessel and the like.

In a cell aggregate or cell mass in suspension culturing, a plane attachment is formed between a cell and a cell. In a cell aggregate or cell mass in suspension culturing, a cell-substratum junction is hardly formed with a culture vessel and the like and, even if it is formed, its contribution is small. In some embodiments, an endogenous cell-substratum junction is present inside the aggregate or cell mass, but a cell-substratum junction is hardly formed with a culture vessel and the like and, even if it is formed, its contribution is small.

The plane attachment between a cell and a cell means that a cell attaches to another cell via planes. More particularly, the plane attachment between a cell and a cell means that, for example, not less than 1%, preferably not less than 3%, more preferably not less than 5%, of the surface area of a cell adheres to the surface of another cell. A surface of a cell can be observed by staining with a reagent (e.g., DiI) that stains membranes, immunostaining of cell adhesion factors (e.g., E-cadherin and N-cadherin).

The culture vessel to be used when performing suspension culturing is not particularly limited as long as it enables "culturing in suspension" and those of ordinary skill in the art can appropriately determine same. Examples of such culture vessel include flask, tissue culture flask, dish, petri dish, tissue culture dish, multidish, microplate, microwell plate, micropore, multiplate, multiwell plate, chamber slide, schale, tube, tray, culture bag, spinner flask, and roller bottle. To enable suspension culturing, these culture vessels are preferably non-cell-adhesive. Useful non-cell-adhesive culture vessels include culture vessels whose surfaces have not undergone an artificial treatment for improving adhesiveness to cells (e.g., surface treatment with extracellular matrix such as basement membrane preparation, laminin, entactin, collagen, and gelatin, or coating treatment with polymer such as polylysine, and polyornithine or positive electric charge treatment and the like), and the like. As a non-cell-adhesive culture vessel, culture vessels whose surfaces have been artificially treated to decrease adhesiveness to the cells (e.g., superhydrophilic treatment with MPC polymer and the like, and protein low adsorption treatment) and the like can be used. Roller culturing using spinner flask, and roller bottle may be performed. The culture surface of the culture vessel may be a flat bottom or may have concaves and convexes.

The medium to be used for culturing cells in the present invention can be prepared from a medium generally used for culturing animal cells as a basal medium. Examples of the basal medium include media that can be used for culturing animal cells such as BME medium, BGJb medium, CMRL1066 medium, Glasgow MEM medium, Improved MEM Zinc Option medium, IMDM medium, Medium199 medium, Eagle MEM medium, αMEM medium, DMEM medium, F-12 medium, DMEM/F-12 medium, IMDM/F12 medium, Ham's medium, RPMI1640 medium, Fischer's medium, and mixed medium thereof.

For culturing pluripotent stem cells, a medium for culturing pluripotent stem cells using the above-mentioned basal medium as the base, preferably a known medium for embryonic stem cells and/or induced pluripotent stem cells, a medium for culturing pluripotent stem cells under feeder free can be used. For example, feeder-free medium such as Essential 8 medium, TeSR medium, mTeSR medium, mTeSR-E8 medium, and StemFit medium can be mentioned.

The "serum-free medium" in the present invention means a medium free of unadjusted or unpurified serum. In the present invention, a medium containing purified blood-derived components and animal tissue-derived components (e.g., growth factor) is also included in a serum-free medium unless unadjusted or unpurified serum is contained therein.

The serum-free medium may contain a serum replacement. Examples of the serum replacement include one appropriately containing albumin, transferrin, fatty acid, collagen precursor, trace element, 2-mercaptoethanol or 3' thiolglycerol, or equivalents of these, and so on. Such serum replacement may be prepared by, for example, the method described in WO98/30679. The serum replacement may be a commercially available product. Examples of such commercially available serum replacement include Knockout™ Serum Replacement (Life Technologies: hereinafter sometimes referred to as KSR), Chemically Defined Lipid Concentrated (manufactured by Life Technologies) and Glutamax™ (manufactured by Life Technologies), B27 (manufactured by Life Technologies), and N2 (manufactured by Life Technologies).

The serum-free medium to be used for suspension culturing may appropriately contain a fatty acid or lipid, amino acid (e.g., non-essential amino acids), vitamin, growth factor, cytokine, antioxidant, 2-mercaptoethanol, pyruvic acid, buffering agent, inorganic salts and so on.

To avoid complicated preparation, a serum-free medium supplemented with an appropriate amount (e.g., about 0.5% to about 30%, preferably about 1% to about 20%) of commercially available KSR (manufactured by Life Technologies) (e.g., medium of 1:1 mixture of F-12 medium and IMDM medium supplemented with 1 x Chemically-defined Lipid concentrated, 5% KSR and 450 µM 1-monothioglycerol) may be used as such serum-free medium. In addition, as a product equivalent to KSR, the medium disclosed in JP-A-2001-508302 can be mentioned.

The "serum-containing medium" in the present invention means a medium containing unadjusted or unpurified serum. The medium may contain a fatty acid, lipid, amino acid (e.g., non-essential amino acids), vitamin, growth factor, cytokine, antioxidant, 2-mercaptoethanol, 1-monothioglycerol, pyruvic acid, buffering agent, inorganic salts and so on. For example, when a pluripotent stem cell is induced to differentiate into a retinal tissue and the like by using a basement membrane preparation such as Matrigel and the like, a serum-containing medium can be used (Cell Stem Cell, 10(6), 771-775 (2012)).

The culturing in the present invention is preferably performed under xeno-free conditions. The "xeno-free" means conditions eliminating components derived from species different from that of the cell to be cultured.

The medium to be used in the present invention is preferably a medium containing chemically determined components (Chemically defined medium; CDM) to avoid contamination with chemically undetermined components.

The "basement membrane-like structure" in the present invention means a thin membrane structure composed of extracellular matrix. The basement membrane is formed on the basal side of epithelial cells in a living body. The components of the basement membrane include type IV collagen, laminin, heparan sulfate proteoglycan (perlecan), entactin/nidogen, cytokine, growth factor and the like. Whether a basement membrane is present in a tissue derived from a living body and in a cell mass prepared by the production method of the present invention is determined by, for example, tissue staining such as PAM staining and the like, and a method such as immunohistochemistry using an antibody against a constituent component of the basement membrane (anti-laminin antibody, anti-type IV collagen antibody, etc.), and the like.

The "basement membrane preparation" in the present invention is one containing a basement membrane constituent component having functions to control epithelial cell-like cell morphology, differentiation, proliferation, motility, functional expression, and the like when desired cells having basement membrane formability are seeded thereon and cultured. For example, when the cells and tissues produced by the present invention are dispersed and further subjected to adhesion culturing, they can be cultured in the presence of a basement membrane preparation. As used herein, the "basement membrane constituent component" refers to an extracellular matrix molecule as a thin membrane present between an epithelial cell layer and an interstitial cell layer and the like in animal tissues. A basement membrane preparation can be produced, for example, by removing cells adhered to the support via a basement membrane and having the ability to form the basement membrane from the support by using a solution having the ability to dissolve the lipids of the cells, an alkaline solution or the like. Examples of the basement membrane preparation include products commercially available as basement membrane products (e.g., Matrigel™ (manufactured by Becton, Dickinson and Company: hereinafter sometimes referred to as Matrigel)) and Geltrex™ (manufactured by Life Technologies), and extracellular matrix molecules known as basement membrane components (e.g., laminin, type-IV collagen, heparan sulfate proteoglycan, and entactin).

In the present invention, a basement membrane preparation such as Matrigel (manufactured by Corning) which is extracted from a tissue or cell of Engelbreth-Holm-Swarm (EHS) mouse sarcoma and the like and solubilized and the like can be used for culturing cells and tissues. Similarly, as a basement membrane component used for cell culturing, human solubilized amniotic membrane (manufactured by Bioresource Application Institute, Co.), human recombinant laminin produced by HEK293 cell (manufactured by BioLamina), human recombinant laminin fragment (manufactured by Nippi, Inc.), and human recombinant vitronectin (manufactured by Thermo Fisher) can also be used. To avoid contamination with components derived from different organism species and to avoid the risk of infections, preferred is a recombinant protein whose components are clear.

In the present invention, the "medium containing a substance X" and "in the presence of a substance X" respectively refer to a medium supplemented with an exogenous substance X or a medium containing an exogenous substance X, and in the presence of an exogenous substance X. The exogenous substance X is distinguished from the endogenous substance X which is the substance X endogenously expressed, secreted or produced by, for example, the cells or tissues present in the medium.

For example, a "medium containing a Sonic hedgehog signal transduction pathway activating substance" is a medium supplemented with an exogenous Sonic hedgehog signal transduction pathway activating substance or a medium containing an exogenous Sonic hedgehog signal transduction pathway activating substance.

In the present invention, a "feeder cell" refers to a cell other than a stem cell that co-exists when culturing the stem cell. Examples of the feeder cells used for culturing pluripotent stem cells while maintaining undifferentiated state include mouse fibroblasts (MEF), human fibroblasts, and SNL cells. As the feeder cells, feeder cells that underwent a growth suppression treatment is preferable. Examples of the growth suppression treatment include treatment with a growth inhibitor (e.g., mitomycin C), and UV irradiation. Feeder cells used for culturing pluripotent stem cells while maintaining undifferentiated state contributes to the maintenance of undifferentiated state of pluripotent stem cell by secretion of humoral factors (preferably factor for maintaining undifferentiated state), or production of scaffolds for cell adhesion (extracellular matrix).

In the present invention, an "aggregate" of cells refers to a clump formed by assembly of cells dispersed in a medium, wherein the cells are adhered to each other. Cell clumps, embryoid bodies, spheres, spheroids, and organoids are also encompassed in the cell aggregates. Preferably, a plane attachment is formed between a cell and a cell in the aggregate of cells. In some embodiments, cells sometimes form a cell-cell junction and/or a cell adhesion, for example, adherence junction, in some or all of the aggregates. The "aggregate" in the present invention specifically includes an aggregate produced in the first step of the below-mentioned "2. Production method of cell mass containing neural cells or neural tissue and nonneural epithelial tissue", which is formed by cells dispersed at the time of the start of the suspension culturing.

In the present invention, "uniformed aggregates" means that the size of each aggregate is constant when a plurality of aggregates are cultured, and that the variance in the length of the maximum diameter is small when the size of the aggregates are evaluated by the length of the maximum diameter. More specifically, it means that not less than 75% of aggregates in the whole aggregate population are within mean ± 100%, preferably mean ± 50%, more preferably mean ± 20%, of the maximum diameter in the population of the cell masses.

In the present invention, to "form uniformed aggregates" means to rapidly aggregate a given number of dispersed cells to form cell aggregates uniform in size, when gathering the cells to form cell aggregates and culturing the aggregates in suspension.

"Dispersion" refers to dividing cells or a tissue into small cell debris (not less than 2 cells and not more than 100 cells, preferably not more than 50 cells) or single cells by a dispersion treatment such as enzymatic treatment, and physical treatment. A given number of dispersed cells is a collection of a certain number of cell debris or single cells.

Examples of the method of dispersing pluripotent stem cells include a mechanical dispersion treatment, a cell dispersion solution treatment, and a cell protecting agent addition treatment. These treatments may be performed in combination. Preferably, a cell dispersion solution treatment is performed and then a mechanical dispersion treatment is performed.

As a method of mechanical dispersion treatment, a pipetting treatment or scraping operation by a scraper can be mentioned.

As a cell dispersion solution to be used for the cell dispersion solution treatment, a solution containing any of enzymes such as trypsin, collagenase, hyaluronidase, elastase, pronase, DNase, and papain, and a chelating agent such as ethylenediaminetetraacetic acid etc. can be mentioned. A commercially available cell dispersion solution such as Accumax (manufactured by Innovative cell technologies) and TrypLE Select (manufactured by Life Technologies) can also be used.

As a cell protecting agent to be used for a cell protector addition treatment, FGF signal transduction pathway activating substance, heparin, ROCK inhibiting substance, serum, or serum replacement can be mentioned. As a preferable cell protecting agent, a ROCK inhibiting substance can be mentioned.

For example, a method for dispersing pluripotent stem cells includes a method involving treating a colony of pluripotent stem cells with a cell dispersion solution (Accumax) in the presence of a ROCK inhibiting substance as a cell protecting agent, and further dispersing them by pipetting.

In the production method of the present invention, it is preferable to form an aggregate of pluripotent stem cells by rapidly gathering the pluripotent stem cells. When an aggregate of pluripotent stem cells is formed in such a manner, an epithelium-like structure can be formed with good reproducibility in the cells induced and differentiated from the formed aggregate. Examples of the experimental operation to form an aggregate include a method involving keeping cells in a small space by using a plate with small wells (e.g., plate with wells having a base area of about 0.1 - 2.0 cm² when calculated in terms of flat bottom), micropore and so on, a method involving aggregating cells by centrifugation for a short time using a small centrifugation tube. As a plate with small wells, for example, 24 well plate (area of about 1.88 cm² when calculated in terms of flat bottom), 48 well plate (area of about 1.0 cm² when calculated in terms of flat bottom), 96 well plate (area of about 0.35 cm² when calculated in terms of flat bottom, inner diameter about 6 - 8 mm), and 384 well plate can be mentioned. Preferred is 96 well plate. As a shape of the plate with small wells, the shape of the bottom surface when the well is seen from above is, for example, polygon, rectangle, ellipse, true circle, preferably true circle. As a shape of the plate with small wells when the well is seen from the side well, the shape of the bottom surface is preferably a structure having high outer circumference and low inner concave, which is, for example, U-bottom, V-bottom or M-bottom, preferably U-bottom or V-bottom, most preferably V-bottom. As a plate with small wells, a cell culture dish (e.g., 60 mm - 150 mm dish, culture flask) with a concave convex, or dent on the bottom surface may also be used. The bottom surface of a plate with small wells is preferably a non-cell-adhesive bottom surface, preferably the aforementioned non-cell-adhesive-coated bottom surface.

Formation of aggregates of pluripotent stem cells, and formation of an epithelium -like structure in each cell forming the aggregate can be determined based on the size and cell number of the aggregate, macroscopic morphology of the aggregate, microscopic morphology by tissue staining analysis and uniformity thereof, expression of differentiation and undifferentiation markers and uniformity thereof, control of expression of differentiation marker and synchronism thereof, reproducibility of differentiation efficiency between aggregates, and so on.

The "tissue" in the present invention refers to a structure of a cell population having a structure in which plural types of cells having different morphologies and properties are sterically arranged in a given pattern.

In the present invention, the "neural tissue" refers to a tissue constituted of neural cells including cerebrum, midbrain, cerebellum, spinal cord, retina, peripheral nerve and the like in the developing stage or adult stage. A neural tissue sometimes forms an epithelial structure (neuroepithelium) having a layer structure, and the amount of neuroepithelium in a neural tissue can be evaluated by bright field observation using an optical microscope.

In the present invention, the "neural cell" refers to a cell other than epidermal lineage cell in a tissue derived from ectoderm. That is, it includes cells such as neural precursor cell, neuron (neuronal cell), glia cell, neural stem cell, neuron precursor cell, glial precursor cell and the like. The neural cell also encompasses cell constituting the below-mentioned retinal tissue (retinal cell), retinal progenitor cell, retinal layer-specific neuron, neural retinal cell, and retinal pigment epithelial cell. The neural cell can be identified by using Nestin, TuJ1, PSA-NCAM, N-cadherin and the like as a marker.

Neuron is a functional cell that forms a neural circuit and contributes to signal transmission, and can be identified by using the expression of immature neuronal markers such as TuJ1, Dcx, and HuC/D and/or mature neuronal cell markers such as Map2, and NeuN as an index.

As glial cell, astrocyte, oligodendrocyte, and Müller glia can be mentioned. As an astrocyte marker, GFAP can be mentioned; as an oligodendrocyte marker, O4 can be mentioned; and as a Müller glia marker, CRALBP and the like can be mentioned.

The neural stem cell is a cell having differentiation potency (multipotency) into neuron and glial cell, and proliferative capacity (sometimes referred to as self-renewal competence) maintaining multipotency. As the neural stem cell marker, Nestin, Sox2, Musashi, Hes family, CD133 etc. can be mentioned; however, these markers are markers for progenitor/precursor cells in general and are not considered neural stem cell-specific markers. The number of neural stem cells can be evaluated by neurosphere assay, and clonal assay.

The neuronal precursor cell is a cell having proliferative capacity, which produces neuron and does not produce glial cell. As a neuronal precursor cell marker, Tbr2, Tα1 etc. can be mentioned. Alternatively, an immature neuronal marker (TuJ1, Dcx, HuC/D)-positive and growth marker (Ki67, pH3, MCM)-positive cell can also be identified as a neuronal precursor cell.

The glial precursor cell is a cell having proliferative capacity, which produces glial cell and does not produce neuron.

The neural precursor cell is an assembly of precursor cells including neural stem cell, neuronal precursor cell and glial precursor cell, and has proliferative capacity and neuron- and glial cell- productivity. The neural precursor cell can be identified using Nestin, GLAST, Sox2, Sox1, Musashi, Pax6 and the like as markers. Alternatively, a neural cell marker-positive and growth marker (Ki67, pH3, MCM)-positive cell can also be identified as a neural precursor cell.

In the present invention, the "retinal tissue" means a retinal tissue in which at least two or more types of cells such as photoreceptor cells, horizontal cells, bipolar cells, amacrin cells, retinal ganglion cells, their progenitor/precursor cells and retinal progenitor cells and so on, which constitute respective retinal layers in retina in vivo, are sterically arranged in layers. Which retinal layer is constituted by each cell can be confirmed by a known method, for example, presence or absence of the expression of a cell marker or the level thereof, and the like.

The "retinal layer" in the present invention means each layer constituting the retina. Specific examples thereof include retinal pigment epithelial layer, photoreceptor cell layer, external limiting membrane, outer nuclear layer, outer plexiform layer, inner nuclear layer, inner plexiform layer, ganglion cell layer, nerve fiber layer and inner limiting membrane.

The "retinal progenitor cell" in the present invention refers to a progenitor cell capable of differentiating into any mature retinal cell including photoreceptor cell, horizontal cell, bipolar cell, amacrine cell, retinal ganglion cell, retinal pigment epithelial cell and the like.

The photoreceptor precursor cell, precursor cell of horizontal cell, precursor cell of bipolar cell, precursor cell of amacrine cell, precursor cell of retinal ganglion cell, and retinal pigment epithelial precursor cell respectively refers to precursor cell committed to differentiate into photoreceptor cell, horizontal cell, bipolar cell, amacrine cell, retinal ganglion cell, and retinal pigment epithelial cell.

In the present invention, the "retinal layer-specific neuron" is a cell constituting a retina layer and is a neuron specific to the retinal layer. Examples of the retinal layer-specific neuron include bipolar cell, retinal ganglion cells, amacrine cell, horizontal cell, photoreceptor, retinal pigment epithelial cell, rod cell and cone cell.

The "retinal cell" in the present invention encompasses the aforementioned retinal progenitor cell and retina layer-specific nerve cell.

Examples of the retinal cell marker include Rx (also referred to as Rax), Aldhla3, and PAX6 expressed in retinal progenitor cell, Nkx2.1 expressed in progenitor cell of hypothalamus neuron but not expressed in retinal progenitor cell, Sox1 expressed in hypothalamus neuroepithelium but not expressed in retina, Crx and Blimp1 expressed in precursor cell of photoreceptor, and the like. Examples of the marker of the retinal layer-specific neuron include Chx10, PKCα and L7 expressed in bipolar cell, TUJI and Brn3 expressed in retinal ganglion cells, Calretinin expressed in amacrine cell, Calbindin expressed in horizontal cell, Rhodopsin and Recoverin expressed in mature photoreceptor, Nrl expressed in rod cell, Rxr-gamma expressed in cone cell, and RPE65 and Mitf expressed in retinal pigment epithelial cell,.

In the present invention, the "nonneural epithelial tissue" refers to a tissue other than the neuroepithelial tissues among the tissues having an epithelial structure. An epithelial tissue can also be formed from any germ layer of ectoderm, mesoderm, endoderm, or nutrition ectoderm. The epithelial tissue includes epithelium, mesothelium, and endothelium. Examples of the tissue included in the nonneural epithelial tissue include epidermis, corneal epithelium, nasal cavity epithelium, mouth cavity epithelium, trachea epithelium, bronchus epithelium, airway epithelium, kidney epithelium, renal cortex epithelium, placenta epithelium and the like.

Epithelial tissues are generally connected by various intercellular junctions, and form tissues having a monolayer or multilayer structure. Confirmation of the presence or absence of such epithelial tissues and quantification of the amount thereof can be performed by observation with an optical microscope or a method such as immunohistochemistry using antibodies against epithelial cell markers (anti-E-Cadherin antibody, anti-N-Cadherin antibody, anti-EpCAM antibody etc.) or the like.

In the present invention, the "epithelial polarity" shows spatially formed bias of the distribution and cellular functions in epithelial cells. For example, corneal epithelial cells are localized in the outermost layer of the eyeball, express apical-specific proteins such as membrane-bound mucins (MUC-1, 4, 16) and the like on the apical side to retain tears, and express basal-specific proteins such as α6 integrin, β1 integrin and the like on the basal side to adhere to the basement membrane.

Whether epithelial polarity is present in a tissue derived from a living body and in a cell mass prepared by the production method of the present invention can be detected by, for example, a method such as immunohistochemistry using Phalloidin, an apical marker (anti-MUC-1 antibody, anti-PKC-zeta antibody etc.) and a basal marker (anti-α6 integrin antibody, anti-β1 integrin antibody etc.) and the like.

Cornea is one kind of nonneural epithelial tissue, and is a transparent watch-glass-like tissue that occupies about 1/6 anterior of the outer layer of the eyeball wall. Examples of the partial structure of the cornea include, but are not limited to, corneal epithelium, Bowman's membrane, corneal stroma, Descemet's membrane, and corneal endothelium,. Cornea is usually composed of five layers consisting of a corneal epithelium, Bowman's membrane, corneal stroma, Descemet's membrane, and corneal endothelium in order from the surface of the body. Induction of cornea, a partial structure thereof, or a precursor tissue thereof can be confirmed by the expression of a marker. Examples of the marker of cornea, a partial structure thereof, or a precursor tissue thereof include pan-cytokeratin (corneal epithelium precursor tissue), cytokeratin 18 (corneal epithelium precursor tissue), cytokeratin 19 (corneal epithelium precursor tissue), EpCAM (corneal epithelium precursor tissue), PDGFR-β (surface layer ectoderm), Six1 (surface layer ectoderm and placode), E-cadherin (corneal epithelium precursor tissue), N-cadherin (corneal epithelial stem cell, precursor tissue), cytokeratin 3 (corneal epithelium), cytokeratin 12 (corneal epithelium), cytokeratin 14 (corneal epithelium), p63 (corneal epithelium), ZO-1 (corneal epithelium), PDGFR-α (corneal stroma, corneal endothelium, or precursor tissue thereof), Pitx2 (precursor tissue of corneal stroma and corneal endothelium), ABCG2 (precursor tissue of corneal stroma and corneal endothelium) and the like. In one embodiment, the precursor tissue of the corneal epithelium contained in the cell mass produced by the method of the present invention is a pan-cytokeratin-positive and E-cadherin-positive epithelial cell layer. In one embodiment, the corneal epithelium contained in the cell mass produced by the method of the present invention is a cytokeratin 3-positive, cytokeratin 12-positive, cytokeratin 14-positive, p63-positive and ZO-1-positive epithelial structure. In one embodiment, the precursor tissue of corneal stroma and corneal endothelium contained in the cell mass produced by the method of the present invention is an aggregate layer of mesenchymal cells. In one embodiment, the aggregate layer of the mesenchymal cells is pan-cytokeratin positive, PDGFR-α positive, or Pitx2 positive and ABCG2 positive. While the corneal stroma and corneal endothelium are both derived from mesenchymal cells, corneal endothelium takes an epithelialized endothelial cell layer-like form. Thus, it is possible to distinguish corneal stroma (or precursor tissue thereof) from corneal endothelium (or precursor tissue thereof) and confirm induction of corneal endothelium or precursor tissue thereof by performing the above-mentioned analysis of marker expression, as well as morphological observation.

In the present invention, the "placode" refers to primordia of organs formed by thickening of a part of epidermal ectoderm mainly in the developmental process of vertebrate. As the tissue derived from placode, crystallin lens, nose, inner ear, trigeminal nerve and the like can be mentioned. As a marker of placode or a precursor tissue thereof (preplacode region), Six1, Six4, Dlx5, Eya2 and the like can be mentioned.

The lens placode is a crystallin lens precursor tissue composed of a thickened epidermal ectoderm cell layer. In the embryonic development, it is formed by the contact of the optic vesicle with the epidermal ectoderm and thickening of the contact region.

The crystallin lens is one of the tissues derived from placode, and is a tissue that plays the role of a lens that refracts light entering the eyeball from outside and focuses to the retina. Examples of the partial structure of crystallin lens include, but are not limited to, crystallin lens epithelium, nucleus of crystallin lens, and crystallin lens capsule,. As the precursor tissue of crystallin lens, lens placode, and lens vesicle can be mentioned.

The lens vesicle is a vesicle formed by invagination of lens placode.

Induction of crystallin lens, a partial structure thereof, or a precursor tissue thereof can be confirmed by the expression of the marker. Examples of the marker of crystallin lens, a partial structure thereof, or a precursor tissue thereof include, but are not limited to, Six1 (surface layer ectoderm and placode), FoxC1 (placode), Emx2 (placode), Sox1 (central nervous system and crystallin lens precursor tissue), FoxE3 (crystallin lens precursor tissue), Prox1 (crystallin lens precursor tissue), L-Maf (crystallin lens precursor tissue), α, β and γ crystallin (crystallin lens) and the like. In one embodiment, the lens placode is an L-Maf positive, thickened epidermal ectoderm cell layer. In one embodiment, the lens vesicle is an L-Maf positive vesicle.

As one embodiment of the cell mass containing neural tissue and nonneural epithelial tissue described below, which is produced by the production method of the present invention, anterior ocular segment tissue can be mentioned. A cell mass containing anterior ocular segment tissue or a partial structure thereof, or a precursor tissue thereof can be obtained by inducing, in the aggregate produced from a pluripotent stem cell, differentiation of pluripotent cells into anterior ocular segment tissue or a partial structure thereof, or precursor tissue thereof. The above-mentioned anterior ocular segment tissue includes cornea tissue which is one kind of nonneural epithelial tissue, crystallin lens which is one kind of placode-derived tissue, and retinal tissue which is one kind of neural tissue.

### 2. Production method of cell mass containing neural cells or neural tissue and nonneural epithelial tissue

The present invention provides a method for producing a cell mass comprising 1) neural cells or neural tissue and 2) nonneural epithelial tissue. In the following, it is also to be referred to as the production method of the present invention.

One embodiment of the production method of the present invention is a method for producing a cell mass containing neural cells or neural tissue and nonneural epithelial tissue, including the following steps (1) and (2):
(1) a first step of suspension-culturing pluripotent stem cells to form a cell aggregate in the presence of a Wnt signal transduction pathway inhibiting substance,
(2) a second step of suspension-culturing the aggregate obtained in the first step in the presence of a BMP signal transduction pathway activating substance, thereby obtaining a cell mass containing 1) neural cells or neural tissue and 2) nonneural epithelial tissue.

A more preferable one embodiment of the production method of the present invention is a method for producing a cell mass containing 1) neural cells or neural tissue and 2) nonneural epithelial tissue, including the following steps (a), (1) and (2) :
(a) step a of maintenance-culturing pluripotent stem cells in the absence of feeder cells and in a medium containing 1) a TGFβ family signal transduction pathway inhibiting substance and/or a Sonic hedgehog signal transduction pathway activating substance, and 2) a factor for maintaining an undifferentiated state,
   (1) a first step of suspension-culturing the pluripotent stem cell, which was maintenance-cultured in step a, to form a cell aggregate in the presence of a Wnt signal transduction pathway inhibiting substance,
   (2) a second step of suspension-culturing the aggregate obtained in the first step in the presence of a BMP signal transduction pathway activating substance, thereby obtaining a cell mass containing 1) neural cells or neural tissue and 2) nonneural epithelial tissue.

### <Step (a)>

The step a of maintenance-culturing pluripotent stem cells in the absence of feeder cells in a medium containing 1) a TGFβ family signal transduction pathway inhibiting substance and/or a Sonic hedgehog signal transduction pathway activating substance, and 2) a factor for maintaining undifferentiated state is explained below.

When pluripotent stem cells are treated with a TGFβ family signal transduction pathway inhibiting substance and/or a Sonic hedgehog signal transduction pathway activating substance in step a, and subjected to suspension culturing in the first step, the condition of the pluripotent stem cells changes, the efficiency of nonneural epithelial tissue formation is improved, the quality of aggregate is improved, differentiation becomes easier, cell death does not occur easily, and a cell aggregate maintaining a densely undifferentiated state of the inside can be produced with high efficiency.

It is preferable to perform step (a) in the absence of feeder cells.

The absence of feeder cells (feeder-free) in the present invention means a condition substantially free of feeder cells (e.g., the ratio of the number of feeder cells relative to the total number of cells is not more than 3%).

The medium to be used in step (a) is not particularly limited as long as it is a medium enabling culturing of pluripotent stem cells to maintain undifferentiated state under feeder-free conditions (feeder-free medium). For example, a medium containing a factor for maintaining an undifferentiated state, and a TGFβ family signal transduction pathway inhibiting substance and/or a Sonic hedgehog signal transduction pathway activating substance can be mentioned.

To enable culturing to maintain undifferentiated state, the medium used in step (a) contains a factor for maintaining undifferentiated state. The factor for maintaining undifferentiated state is not particularly limited as long as it is a substance having an action to suppress differentiation of pluripotent stem cells. Examples of the factor for maintaining undifferentiated state widely used by those of ordinary skill in the art include an FGF signal transduction pathway activating substance, a TGFβ family signal transduction pathway activating substance, and insulin in the case of primed pluripotent stem cells (e.g., human ES cells, human iPS cells). As the FGF signal transduction pathway activating substance, fibroblast growth factors (e.g., bFGF, FGF4, FGF8) can be specifically mentioned. As the TGFβ family signal transduction pathway activating substance, a TGFβ signal transduction pathway activating substance, a Nodal/Activin signal transduction pathway activating substance can be mentioned. As the TGFβ signal transduction pathway activating substance, for example, TGFβ1, TGFβ2 can be mentioned. As the Nodal/Activin signal transduction pathway activating substance, for example, Nodal, Activin A, Activin B can be mentioned. When human pluripotent stem cells (human ES cells, human iPS cells) are cultured, the medium in step (a) preferably contains bFGF as a factor for maintaining undifferentiated state.

The factor for maintaining undifferentiated state to be used in the present invention is generally a factor for maintaining undifferentiated state of mammals. The mammals are, for example, those mentioned above. Since the factor for maintaining undifferentiated state may have cross-reactivity among mammal species, a factor for maintaining undifferentiated state of any mammal may also be used as long as the undifferentiated state of the pluripotent stem cells to be cultured can be maintained. Preferably, a factor for maintaining undifferentiated state of a mammal of the same species as the cells to be cultured is used. For example, for the culturing of human pluripotent stem cells, human factor for maintaining undifferentiated states (e.g., bFGF, FGF4, FGF8, EGF, Nodal, Activin A, Activin B, TGFβ 1, and TGFβ 2) are used. Here, the "human protein X" means that protein X (a factor for maintaining undifferentiated state etc.) has the amino acid sequence of protein X naturally expressed in human in vivo.

The factor for maintaining undifferentiated state to be used in the present invention is preferably isolated.

Being "isolated" means that an operation to remove factors other than the intended component or cell has been performed, and the component or cell is no longer in a naturally occurring state. Therefore, "isolated protein X" does not include an endogenous protein X produced from the cells or tissues to be cultured, and contained in a cell or tissue or in the medium. The purity of the "isolated protein X" (percentage of the weight of protein X to the total protein weight) is generally not less than 70%, preferably not less than 80%, more preferably not less than 90%, further preferably not less than 99%, most preferably 100%.

In one embodiment, the present invention comprises a step of providing an isolated factor for maintaining undifferentiated state. In one embodiment, it includes a step of exogenously adding an isolated factor for maintaining undifferentiated state to a medium used in step (a). Alternatively, a factor for maintaining undifferentiated state may be added in advance to a medium to be used in step (a).

The concentration of the factor for maintaining undifferentiated state in the medium to be used in step (a) is a concentration capable of maintaining the undifferentiated state of the pluripotent stem cells to be cultured, and can be appropriately determined by those of ordinary skill in the art. For example, when bFGF is used as a factor for maintaining undifferentiated state in the absence of feeder cells, the concentration thereof is generally about 4 ng/mL - 500 ng/mL, preferably about 10 ng/mL - about 200 ng/mL, more preferably about 30 ng/mL - 150 ng/mL.

As the feeder-free medium, many synthetic media have been developed and are commercially available and, for example, Essential 8 medium can be mentioned. Essential 8 medium is DMEM/F12 medium containing L-ascorbic acid-2-phosphate magnesium (64 mg/l), sodium selenium (14 µg/l), insulin (19.4 mg/l), NaHCO₃ (543 mg/l), transferrin (10.7 mg/l), bFGF (100 ng/mL), and a TGFβ family signal transduction pathway activating substance (TGFβ 1 (2 ng/mL) or Nodal (100 ng/mL)) as additives (Nature Methods, 8, 424-429 (2011)). Examples of the commercially available feeder-free medium include Essential 8 (manufactured by Life Technologies), S-medium (manufactured by DS Pharma Biomedical), StemPro (manufactured by Life Technologies), hESF9, mTeSR1 (manufactured by STEMCELL Technologies), mTeSR2 (manufactured by STEMCELL Technologies), and TeSR-E8 (manufactured by STEMCELL Technologies). In addition to these, StemFit (manufactured by Ajinomoto Co., Inc.) can be mentioned as the feeder-free medium. The present invention can be performed conveniently by using these in the above-mentioned step (a). The StemFit medium contains bFGF as a component for maintaining an undifferentiated state as described in Nakagawa et al., Scientific Reports, 4, 3594, 2014.

While the medium used for step (a) may be a serum-containing medium or a serum-free medium, it is preferably a serum-free medium, to avoid contamination with chemically-undefined components.

To avoid contamination with a chemically-undefined component, a medium to be used for step (a) is preferably a medium whose components are chemically-defined.

In step (a), the pluripotent stem cells may be cultured under any conditions of suspension culturing and adhesion culturing, preferably adhesion culturing.

For culturing pluripotent stem cells under feeder-free conditions in step (a), the aforementioned feeder-free medium can be used as a medium.

For culturing pluripotent stem cells under feeder-free conditions in step (a), an appropriate matrix may be used as a scaffold to provide a scaffold in stead of the feeder cells to the pluripotent stem cell. The pluripotent stem cells are subjected to adhesion culturing in a cell container whose surface is coated with a matrix as a scaffold.

As a matrix available as a scaffold, laminin (Nat Biotechnol 28, 611-615 (2010)), laminin fragment (Nat Commun 3, 1236 (2012)), basement membrane preparation (Nat Biotechnol 19, 971-974 (2001)), gelatin, collagen, heparan sulfate proteoglycan, entactin, vitronectin and the like can be mentioned.

"Laminin" is a heterotrimer molecule consisting of α, β, γ chains and an extracellular matrix protein containing isoforms having different subunit chain compositions. Specifically, laminin has about 15 kinds of isoforms based on the combinations of heterotrimers with 5 kinds of α chains, 4 kinds of β chains and 3 kinds of γ chains. The name of laminin is determined by combining respective numbers of α chain (α1 - α5), β chain (β1 - β4) and γ chain (γ1 - γ4). For example, a laminin having a combination of α5 chain, β1 chain, γ1 chain is named laminin 511. In the present invention, laminin 511 is preferably used (Nat Biotechnol 28, 611-615 (2010)).

A laminin fragment to be used in the present invention is not particularly limited as long as it has adhesiveness to pluripotent stem cells and enables maintenance culturing of pluripotent stem cell under feeder-free conditions, and is preferably E8 fragment. Laminin E8 fragment was identified as a fragment with strong cell adhesion activity among the fragments obtained by digestion of laminin 511 with elastase (EMBO J., 3:1463-1468, 1984, J. Cell Biol., 105:589-598, 1987). In the present invention, E8 fragment of laminin 511 is preferably used (Nat Commun 3, 1236 (2012), Scientific Reports 4, 3549 (2014)). The laminin E8 fragment to be used in the present invention is not required to be an elastase digestion product of laminin and may be a recombinant. Alternatively, it may be produced by a gene recombinant animal (Bombyx mori etc.). To avoid contamination of unidentified components, a recombinant laminin fragment is preferably used in the present invention. An E8 fragment of laminin 511 is commercially available and can be purchased from, for example, Nippi, Inc. and the like.

To avoid contamination with unidentified component, the laminin or laminin fragment to be used in the present invention is preferably isolated.

Preferably, in the culturing of pluripotent stem cells under feeder-free conditions in step (a), the human pluripotent stem cells are cultured in an adhered state in a cell container with surface coated with isolated laminin 511 or E8 fragment of laminin 511 (most preferably, E8 fragment of laminin 511).

While the period for the culturing of pluripotent stem cells in step (a) is not particularly limited as long as the effect of improving the quality of the aggregate formed in subsequent step (1) can be achieved, it is generally 0.5 - 144 hr, preferably 2 - 96 hr, more preferably 6 - 48 hr, further preferably 12 - 48 hr, further more preferably 18 - 28 hr (e.g., 24 hr).

That is, step (a) is started 0.5 - 144 hr (preferably, 18 - 28 hr) before the start of step (1), and step (1) is continuously performed on completion of step (a).

The culturing conditions such as culturing temperature, and CO₂ concentration in step (a) can be appropriately determined. The culturing temperature is, for example, about 30°C to about 40°C, preferably about 37°C. The CO₂ concentration is, for example, about 1% to about 10%, preferably about 5%.

In one preferable embodiment, human pluripotent stem cells (e.g., human ES cell, human iPS cells) are cultured in an adhered state in the absence of feeder cells and in a serum-free medium containing bFGF. The adhesion culturing is preferably performed in a cell container with surface coated with laminin 511, E8 fragment of laminin 511 or vitronectin. The adhesion culturing is preferably performed using StemFit medium as a feeder-free medium.

In one preferable embodiment, human pluripotent stem cells (e.g., human ES cell, human iPS cells) are cultured in suspension in the absence of feeder cells and in a serum-free medium containing bFGF. In the suspension-culturing, human pluripotent stem cells may form an aggregate of human pluripotent stem cells.

The Sonic hedgehog (hereinafter sometimes referred to as Shh) signal transduction pathway activating substance is a substance capable of enhancing signal transduction mediated by Shh. Examples of the Shh signal transduction pathway activating substance include proteins belonging to the Hedgehog family (e.g., Shh, Ihh), Shh receptor, Shh receptor agonist, Smo agonist, Purmorphamine, GSA-10, Hh-Ag1.5, and 20(S)-Hydroxycholesterol or SAG (Smoothened Agonist; N-Methyl-N'-(3-pyridinylbenzyl)-N'-(3-chlorobenzo[b]thiophene-2-carbonyl)-1,4-diaminocyclohexane). The Shh signal transduction pathway activating substance is preferably SAG.

The concentration of the Shh transduction pathway activating substance in the medium can be appropriately determined to fall within a range capable of achieving the aforementioned effects. In step (a), SAG is generally used at a concentration of about 1 nM - about 2000 nM, preferably about 10 nM - about 1000 nM, more preferably about 10 nM - about 700 nM, further preferably about 50 nM - about 700 nM, particularly preferably about 100 nM - about 600 nM, most preferably about 100 nM - about 500 nM. When an Shh signal transduction pathway activating substance other than SAG is used, it is desirably used at a concentration that shows Shh signal transduction promoting activity equivalent to that of SAG at the aforementioned concentration. Sonic hedgehog signal transduction promoting activity can be determined by a method well known to those of ordinary skill in the art, for example, reporter gene assay focusing on the expression of Gli1 gene (Oncogene (2007) 26, 5163-5168). For example, as the Shh transduction pathway activating substance having Sonic hedgehog signal transduction promoting activity corresponding to that of 10 nM to 700 nM SAG, 20 nM to 2 µM of Purmorphamine, 20 nM to 3 pM of GSA-10, 10 nM to 1 µM of Hh-Ag1.5 and the like can be mentioned.

The TGFβ family signal transduction pathway (i.e., TGFβ superfamily signal transduction pathway) is a signal transduction pathway intracellularly transduced by Smad family with TGFβ, Nodal/Activin or BMP as a ligand.

The TGFβ family signal transduction pathway inhibiting substance is a substance that inhibits TGFβ family signal transduction pathway, that is, a signal transduction pathway transduced by the Smad family. Specifically, a TGFβ signal transduction pathway inhibiting substance, a Nodal/Activin signal transduction pathway inhibiting substance and a BMP signal transduction pathway inhibiting substance can be mentioned. As the TGFβ family signal transduction pathway inhibiting substance, a TGFβ signal transduction pathway inhibiting substance is preferable.

The TGFβ signal transduction pathway inhibiting substance is not particularly limited as long as it is a substance inhibiting a signal transduction pathway caused by TGFβ, and may be any of nucleic acid, protein and low-molecular organic compound. As the substance, for example, a substance directly acting on TGFβ (e.g., protein, antibody, and aptamer), a substance suppressing expression of gene encoding TGFβ (e.g., antisense oligonucleotide, and siRNA), a substance that inhibits the binding of TGFβ receptor and TGFβ, and a substance that inhibits physiological activity caused by signal transduction by the TGFβ receptor (e.g., TGFβ receptor inhibitor, and Smad inhibitor) can be mentioned. As a protein known as a TGFβ signal transduction pathway inhibiting substance, Lefty and the like can be mentioned.

As a TGFβ signal transduction pathway inhibiting substance, compounds well known to those of ordinary skill in the art can be used. Specifically, Alk5/TGFβR1 inhibitors such as SB431542 (sometimes to be abbreviated as SB431 in the present specification), SB505124, SB525334, LY2157299, LY2109761, GW788388, LY364947, SD-208, EW-7197, A 83-01, and RepSox, and SMAD3 inhibitors such as SIS3 and the like can be mentioned. SB431542 (4-(5-benzol[1,3]dioxol-5-yl-4-pyridin-2-yl-1H-imidazol-2-yl)-benzamide) and A-83-01 (3-(6-methyl-2-pyridinyl)-N-phenyl-4-(4-quinolinyl)-1H-pyrazole-1-carbothioamide) are compounds known as inhibitors of TGFβ receptor (ALK5) and Activin receptor (ALK4/7) (i.e., TGFβR inhibitor). SIS3 is a TGFβ signal transduction pathway inhibiting substance that inhibits phosphorylation of SMAD3 which is an intracellular signal transduction factor under the control of TGFβ receptor. The TGFβ signal transduction pathway inhibiting substance used in the present invention is preferably SB431542 or A-83-01.

The concentration of a TGFβ signal transduction pathway inhibiting substance in the medium can be appropriately determined as long as the aforementioned effect can be achieved. When SB431542 is used as the TGFβ transduction pathway inhibiting substance in step (a), it is typically used at a concentration of about 1 nM - about 100 µM, preferably about 10 nM - about 50 µM, more preferably about 100 nM - about 50 µM, further preferably about 1 µM - about 10 µM. When a TGFβ signal transduction pathway inhibiting substance other than SB431542 is used, it is desirably used at a concentration that shows TGFβ signal transduction pathway inhibiting activity equivalent to that of SB431542 at the aforementioned concentration.

### <Step (1)>

The first step of suspension-culturing pluripotent stem cells maintained under undifferentiation conditions, preferably pluripotent stem cell obtained in step (a), to form a cell aggregate in the presence of a Wnt signal transduction pathway inhibiting substance is explained.

The Wnt signal transduction pathway is a signal transduction pathway that uses a Wnt family protein as a ligand and mainly uses Frizzled as a receptor. Examples of the signal pathway include the classical Wnt pathway transmitted by β-Catenin (Canonical Wnt pathway), as well as a β-catenin-independent non-classical Wnt pathway (Non-Canonical Wnt pathway) and the like. The Non-Canonical Wnt pathway includes Planar Cell Polarity (PCP) pathway, Wnt/Calcium pathway, Wnt-RAP1 pathway, Wnt-Ror2 pathway, Wnt-PKA pathway, Wnt-GSK3MT pathway, Wnt-aPKC pathway, Wnt-RYK pathway, and Wnt-mTOR pathway. In the Non-Canonical Wnt pathway, a common signal transduction factor which is also activated in other signaling pathways other than Wnt is present. In the present invention, such factors are also considered the constitution factors of the Wnt signal transduction pathway and inhibiting substances of the factors are also included in the Wnt signal transduction pathway inhibiting substance.

In the present invention, the Wnt signal transduction pathway inhibiting substance is not limited as long as it can suppress signal transduction induced by Wnt family proteins. It may be any of nucleic acid, protein and low-molecular organic compound. Examples of the substance include a substance that inhibits Wnt processing and extracellular secretion, a substance that directly acts on Wnt (e.g., antibody, and aptamer), a substance that suppresses expression of a gene encoding Wnt (e.g., antisense oligonucleotide, and siRNA), a substance that suppresses binding of Wnt receptor and Wnt, and a substance that suppresses physiological activity caused by signal transduction by Wnt receptor. As a protein known as a Wnt signal transduction pathway inhibiting substance, proteins belonging to secreted Frizzled Related Protein (sFRP) class (sFRP1 to 5, Wnt inhibitory Factor-1 (WIF-1), Cerberus), proteins belonging to Dickkopf (Dkk) class (Dkk1 to 4, Kremen) and the like can be mentioned.

As the Wnt signal transduction pathway inhibiting substance, a compound well known to those of ordinary skill in the art can be used. As the Wnt signal transduction pathway inhibiting substance, for example, Porcupine inhibitor (to be also referred to as PORCN inhibitor), Frizzled inhibitor, Dvl inhibitor, Tankyrase inhibitor (to be also referred to as TANK inhibitor), casein kinase 1 inhibitor, catenin responsive transcription inhibitor, p300 inhibitor, CBP inhibitor, and BCL-9 inhibitor (Am J Cancer Res. 2015; 5(8): 2344-2360) can be mentioned. While the action mechanism has not been reported, KY 02111 and KY03-I can be recited as the Wnt signal transduction pathway inhibiting substance.

As the PORCN inhibitor, for example, IWP-2, IWP-3, IWP-4, IWP-L6, IWP-12, LGK-974, ETC-159, GNF-6231, and Wnt-C59 can be mentioned.

As the TANK inhibitor, for example, IWR1-endo, XAV939, MN-64, WIKI4, TC-E 5001, JW 55, and AZ6102 can be mentioned.

The Wnt signal transduction pathway inhibiting substance to be used in the present invention is preferably PORCN inhibitor, TANK inhibitor or KY 02111, more preferably PORCN inhibitor.

The PORCN inhibitor to be used in the present invention is preferably IWP-2 or Wnt-C59. The TANK inhibitor to be used in the present invention is preferably XAV939.

The Wnt signal transduction pathway inhibiting substance to be used in the present invention also preferably has inhibiting activity on Non-Canonical Wnt pathway. As a Wnt signal transduction pathway inhibiting substance having inhibiting activity on Non-Canonical Wnt pathway, for example, PORCN inhibitor, anti-Frizzled antibody, and Box5 peptide can be mentioned. It is known that Porcupine is involved in lipid modification of Canonical Wnt pathway ligands Wnt1, Wnt3a and the like, as well as Non-Canonical Wnt pathway ligands Wnt5a, Wnt5b, Wnt11 (Dev Biol. 2012 Jan 15; 361(2):392-402., Biochem J. 2007 Mar 15; 402(Pt 3): 515-523.), and PORCN inhibitor inhibits both the Canonical Wnt pathway and Non-Canonical Wnt pathway.

The concentration of the Wnt signal transduction pathway inhibiting substance in the medium can be appropriately determined to fall within a range capable of achieving the aforementioned effects. For example, when IWP-2 which is one kind of PORCN inhibitor is used as the Wnt signal transduction pathway inhibiting substance, the concentration thereof is typically about 0.01 pM - about 30 µM, preferably about 0.1 pM - about 30 µM, more preferably about 2 pM. When Wnt-C59 which is one kind of PORCN inhibitor is used, the concentration thereof is typically about 1 pM - about 30 µM, preferably about 100 pM - about 30 µM, more preferably about 1 nM - about 1 µM. When XAV939 which is one kind of TANK inhibitor is used, the concentration thereof is typically about 0.01 pM - about 30 µM, preferably about 0.1 pM - about 30 µM, more preferably about 1 µM. When KY 02111 is used, the concentration thereof is typically about 0.01 pM - about 30 µM, preferably about 0.1 pM - about 30 µM, more preferably about 5 µM.

In step (1), the suspension culturing is preferably performed in the presence of a Wnt signal transduction pathway inhibiting substance and a TGFβ signal transduction pathway inhibiting substance.

As a TGFβ signal transduction pathway inhibiting substance to be used in step (1), those similar to the ones exemplified in step (a) can be used. The TGFβ signal transduction pathway inhibiting substances in step (a) and step (1) may be the same or different, preferably the same.

The concentration of the TGFβ signal transduction pathway inhibiting substance in the medium can be appropriately determined to fall within a range capable of achieving the aforementioned effects. When SB431542 is used as the TGFβ signal transduction pathway inhibiting substance, it is typically used at a concentration of about 1 nM - about 100 µM, preferably about 10 nM - about 50 µM, more preferably about 100 nM - about 50 µM, further preferably about 500 nM - about 10 µM. When a TGFβ signal transduction pathway inhibiting substance other than SB431542 is used, it is desirably used at a concentration showing a TGFβ signal transduction pathway inhibiting activity equivalent to that of SB431542 at the aforementioned concentration.

The medium used in step (1) is not particularly limited as long as it is as described in the above-mentioned definition. The medium to be used in step (1) may be a serum-containing medium or serum-free medium. To avoid contamination of chemically-undefined components, a serum-free medium is preferably used in the present invention. To avoid complicated preparation, for example, a serum-free medium supplemented with an appropriate amount of a commercially available serum replacement such as KSR and so on (e.g., medium of 1:1 mixture of IMDM and F-12, which is supplemented with 5% KSR, 450 µM 1-monothioglycerol and 1x Chemically Defined Lipid Concentrate, or GMEM medium supplemented with 5% - 20% KSR, NEAA, pyruvic acid, 2-mercaptoethanol) is preferably used. The amount of KSR to be added to a serum-free medium in the case of human ES cell is generally about 1% to about 30%, preferably about 2% to about 20%.

For formation of an aggregate in step (1), a dispersing operation of the pluripotent stem cells obtained in step (a) is preferably first performed. The "dispersed cells" obtained by the dispersing operation refers to a state where, for example, not less than 70% of cells are single cells and not more than 30% of cells are clumps of 2 - 50 cells. Preferably, as the dispersed cells, a state where not less than 80% of cells are single cells, and not more than 20% of cells are clumps of 2 - 50 cells can be mentioned. The dispersed cells refer to a state almost free of mutual adhesion of cells (e.g., plane attachment). In some embodiments, dispersed cells refer to a state almost free of cell-cell junction (e.g., adhesive bond).

A dispersion operation of the pluripotent stem cells obtained in step (a) may contain the above-mentioned mechanical dispersion treatment, cell dispersion solution treatment, and cell protecting agent addition treatment. These treatments may be performed in combination. Preferably, a cell dispersion solution treatment is performed simultaneously with a cell protecting agent addition treatment and then a mechanical dispersion treatment is performed.

As a cell protecting agent to be used for the cell protecting agent addition treatment, an FGF signal transduction pathway activating substance, heparin, ROCK inhibiting substance, myosin inhibiting substance, serum, or serum replacement can be mentioned. As a preferable cell protecting agent, a ROCK inhibiting substance can be mentioned. To suppress cell death of pluripotent stem cells (particularly, human pluripotent stem cells) induced by dispersion, a Rho-associated coiled-coil kinase (ROCK) inhibiting substance may be added from the start of the first step culturing. As a ROCK inhibiting substance, Y-27632, Fasudil (HA1077), H-1152, HA-100 and the like can be mentioned. Alternatively, a cell protecting agent after preparation can also be used. Examples of the cell protecting agent after preparation include RevitaCell Supplement (manufactured by Thermo Fisher Scientific), and CloneR (manufactured by Stemcell Technologies).

As a cell dispersion solution to be used for the cell dispersion solution treatment, a solution containing any of enzymes such as trypsin, collagenase, hyaluronidase, elastase, pronase, DNase, papain and so on, and a chelating agent such as ethylenediaminetetraacetic acid and so on can be mentioned. A commercially available cell dispersion solution such as TrypLE Select (manufactured by Thermo Fisher Scientific), TrypLE Express (manufactured by Thermo Fisher Scientific), Accumax (manufactured by Innovative Cell Technologies) can also be used.

As a method of mechanical dispersion treatment, a pipetting treatment or scraping by a scraper can be mentioned. The dispersed cells are suspended in the above-mentioned medium.

Then, a suspension of the dispersed pluripotent stem cells is seeded in the above-mentioned culture vessel, and the dispersed pluripotent stem cells are cultured under a condition non-adhesive to the culture vessel, whereby plural cells are gathered to form an aggregate.

In this case, plural cell aggregates may be simultaneously formed in one culture vessel by seeding the dispersed pluripotent stem cells in a comparatively large culture vessel such as a 10 cm dish. To prevent easy occurrence of size dispersion of each aggregate, for example, a given amount of the dispersed pluripotent stem cells are placed in each well of a multiwell plate (U-bottom, V-bottom) such as a 96-well microplate, and static culturing is performed, whereby the cells rapidly aggregate to preferably form one aggregate in each well. The aggregates formed in each well are recovered from plural wells, whereby a population of uniformed aggregates can be obtained.

In step (1), to avoid complicated preparation of the aggregate, a three-dimensional cell culture container permitting exchange of the medium of the whole plate while aggregates are contained in each well may also be used. Examples of the three-dimensional cell culture container include PrimeSurface 96 Slit well plate (manufactured by SUMITOMO BAKELITE CO., LTD.) and the like.

The concentration of the pluripotent stem cells in step (1) can be appropriately set so that cell aggregates can be more uniformly and efficiently formed. For example, when human pluripotent stem cells (e.g., human ES cell, human iPS cell obtained in step (a))) are cultured in suspension using a 96-well microwell plate, a liquid prepared to achieve generally about 1 x 10³ to about 1 x 10⁵ cells, preferably about 3 x 10³ to about 5 x 10⁴ cells, more preferably about 4 x 10³ to about 2 x 10⁴ cells, further preferably about 4 x 10³ to about 1.6 x 10⁴ cells, particularly preferably about 8 x 10³ to about 1.2 x 10⁴ cells, per well is added to the wells, and the plate is left to stand to form aggregates.

The culturing conditions such as culturing temperature, CO₂ concentration and so on in step (1) can be appropriately determined. The culturing temperature is, for example, about 30°C to about 40°C, preferably about 37°C. The CO₂ concentration is, for example, about 1% to about 10%, preferably about 5%.

In step (1), when a medium change operation is performed, for example, it can be performed by an operation to add a fresh medium without discarding the existing medium (medium addition operation), an operation to discard about a half amount of the existing medium (about 30 - 90%, for example, about 40 - 60% of the volume of the existing medium) and add about a half amount of a fresh medium (about 30 - 90%, for example, about 40 - 60% of the volume of the existing medium) (half-medium change operation), and an operation to discard about the whole amount of the existing medium (not less than 90% of the volume of the existing medium) and add about the whole amount of a fresh medium (not less than 90% of the volume of the existing medium) (full- medium change operation) and the like.

When a particular component (e.g., differentiation-inducing factor) is added at a certain time point, for example, an operation to calculate the final concentration, to discard about a half amount of the existing medium, and to add about a half amount of a fresh medium containing a particular component at a concentration higher than the final concentration (half amount medium change operation) may be performed.

When the concentration of a component contained in the existing medium is to be decreased by dilution at a certain time point, for example, the medium change operation may be performed plural times per day, preferably plural times (e.g., 2 - 3 times) within 1 hr. Also, when the concentration of a component contained in the existing medium is to be decreased by dilution at a certain time point, the cell or aggregate may be transferred to another culture container.

While the tool used for the medium change operation is not particularly limited, for example, pipetter, pipetteman, multichannel pipetteman, and continuous dispenser, can be mentioned. For example, when a 96 well plate is used as a culture container, a multichannel pipetteman may be used.

The period for suspension culturing necessary for forming a cell aggregate can be determined as appropriate according to the pluripotent stem cell to be used, so that the cells can be aggregated uniformly. To form uniformed cell aggregates, it is desirably as short as possible. The steps for the dispersed cells to form cell aggregates can be divided into a step for gathering cells, and a step for forming cell aggregates from the gathered cells. In a step of seeding the dispersed cells (i.e., at the time of the start of suspension culturing) to allow for gathering of the cells in case of human pluripotent stem cell (e.g., human ES cell, human iPS cell), for example, the gathered cells are formed preferably within about 24 hr, more preferably within about 12 hr. In the step of seeding the dispersed cells (i.e., at the time of the start of suspension culturing) to allow for forming a cell aggregate in the case of human pluripotent stem cells (e.g., human ES cell, human iPS cells), the aggregate is formed, for example, preferably within about 72 hr, more preferably within about 48 hr. The period for cell aggregate formation can be appropriately adjusted by controlling the tools for aggregating the cells, centrifugation conditions and so on.

Formation of cell aggregates can be determined based on the size and cell number of the aggregates, macroscopic morphology, microscopic morphology and uniformity thereof by tissue staining analysis, expression of markers for differentiation and undifferentiated state and uniformity thereof, control of expression of differentiation marker and synchronism thereof, reproducibility of differentiation efficiency between the aggregates, and so on.

After aggregate formation, the aggregate may be continuously cultured as it is. The period for suspension culturing in step (1) is generally about 8 hr - 6 days, preferably about 12 hr - 48 hr.

### <step (2)>

The second step in which the cell aggregate obtained in step (1) is cultured in suspension in the presence of a BMP signal transduction pathway activating substance to obtain a cell mass containing 1) neural cells or neural tissue and 2) nonneural epithelial tissue is explained below.

The BMP signal transduction pathway activating substance is a substance capable of enhancing signal transduction mediated by BMP. Examples of the BMP signal transduction pathway activating substance include BMP proteins such as BMP2, BMP4, and BMP7, GDF proteins such as GDF7, anti-BMP receptor antibody, BMP partial peptide and so on.

BMP2 protein and BMP4 protein are available from, for example, R&D Systems, BMP7 protein is available from Biolegend, and GDF7 protein is available from, for example, Wako Pure Chemical Industries, Ltd. The BMP signal transduction pathway activating substance is preferably BMP4.

The concentration of the BMP signal transduction pathway activating substance in the medium can be appropriately determined to fall within a range capable of affording the aforementioned effects. When BMP4 is used as a BMP signal transduction pathway activating substance, it is generally used at a concentration of about 1 pM - about 100 nM, preferably about 10 pM - about 50 nM, more preferably about 100 pM - about 25 nM, further preferably about 250 pM - about 10 nM. When a BMP signal transduction pathway activating substance other than BMP4 is used, it is desirably used at a concentration that shows BMP signal transduction pathway promoting activity equivalent to that of BMP4 at the aforementioned concentration.

The medium used in step (2) is not particularly limited as long as it contains a BMP signal transduction pathway activating substance. The medium to be used in step (2) may be a serum-containing medium or serum-free medium. To avoid contamination of chemically-undefined components, a serum-free medium is preferably used in the present invention. To avoid complicated preparation, for example, a serum-free medium supplemented with an appropriate amount of a commercially available serum replacement such as KSR and so on (e.g., medium of 1:1 mixture of IMDM and F-12, which is supplemented with 5% KSR, 450 µM 1-monothioglycerol and 1x Chemically Defined Lipid Concentrate, or GMEM medium supplemented with 5% - 20% KSR, NEAA, pyruvic acid, 2-mercaptoethanol) is preferably used. The amount of KSR to be added to a serum-free medium in the case of human ES cell is generally about 1% to about 30%, preferably about 2% to about 20%. In the second step, the pluripotent stem cell aggregate obtained in the first step is cultured in suspension in a medium (preferably serum-free medium) containing a BMP signal transduction pathway activating substance to form a cell mass, whereby the quality of the cell mass is improved. To be specific, a cell mass containing 1) neural cells or neural tissue and 2) nonneural epithelial tissue, wherein 1) neural cells or neural tissue (preferably not less than 30% of the surface thereof) is coated on 2) nonneural epithelial tissue, more preferably not less than 30 µm of a space is formed between 1) neural cells or neural tissue and 2) nonneural epithelial tissue can be formed with high efficiency.

It is preferable to perform step (2) without adding a Sonic hedgehog signal transduction pathway activating substance. Step (2) is preferably performed substantially in the absence of a Sonic hedgehog signal transduction pathway activating substance. Step (2) may also be performed in the presence of a TGFβ signal transduction pathway inhibiting substance.

### 3. Cell mass containing neural cells or neural tissue, and nonneural epithelial tissue

The present invention provides a cell mass containing 1) neural cells or neural tissue and 2) nonneural epithelial tissue, preferably a cell mass characterized in that 1) neural cells or neural tissue are(is) coated with 2) nonneural epithelial tissue (preferably not less than 30% of the surface of 1) is coated with 2)). In the following, it is also referred to as the cell mass of the present invention. The cell mass of the present invention can be preferably produced by the above-mentioned production method of the present invention.

In the cell mass of the present invention, 1) neural cells or neural tissue are(is) preferably cells or tissue of the central nervous system, or precursor tissue thereof and, as the cell or tissue of the central nervous system, retina, cerebral cortex, diencephalon (e.g., hypothalamus) and the cells derived from such tissues can be mentioned.

In the cell mass of the present invention, 2) nonneural epithelial tissue preferably has epithelial cell polarity and more preferably has a basement membrane-like structure between 1) neural cells or neural tissue.

The nonneural epithelial tissue is preferable pseudostratified epithelium or stratified epithelium. The nonneural epithelial tissue is specifically, for example, epidermis or precursor tissue thereof, cornea or precursor tissue thereof, and oral epithelium or precursor tissue thereof, more preferably cornea or precursor tissue thereof.

In the cell mass of the present invention, an embodiment in which a part of the 2) nonneural epithelial tissue is placode or placode-derived tissue is also preferable. As the placode, cranial placode can be mentioned and as the placode-derived tissue, crystallin lens, inner ear tissue, and trigeminal nerve can be mentioned.

In the cell mass of the present invention, a space of not less than 30 µm, preferably not less than 40 µm, more preferably not less than 50 µm, typically 30 - 1000 µm, may be formed between 1) neural cells or neural tissue and 2) nonneural epithelial tissue. When such space is formed between 1) and 2), nonneural epithelial tissue (e.g., cornea) existing outside can be separated and collected from the neural cells or neural tissue existing inside. Thus, it can be provided as a more accurate transplant material.

Furthermore, tissue (e.g., tissue composed of neural crest-derived cells, tissue composed of mesenchymal cells) may be formed between 1) neural cells or neural tissue and 2) nonneural epithelial tissue.

### 4. Production method of nonneural epithelial tissue sheet

The present invention provides a production method of a nonneural epithelial tissue sheet.

One embodiment of the production method of a nonneural epithelial tissue sheet of the present invention is a production method of a nonneural epithelial tissue sheet including the following steps (1) - (4):
(1) a first step of suspension-culturing pluripotent stem cells to form a cell aggregate in the presence of a Wnt signal transduction pathway inhibiting substance,
(2) a second step of suspension-culturing the aggregate obtained in the first step in the presence of a BMP signal transduction pathway activating substance, thereby obtaining a cell mass containing 1) neural cells or neural tissue and 2) nonneural epithelial tissue,
(3) a third step of collecting 2) nonneural epithelial tissue from the cell mass obtained in the second step,
(4) a fourth step of dispersing the 2) nonneural epithelial tissue obtained in the third step and culturing same on a flat plane, thereby obtaining a nonneural epithelial tissue sheet.

Another embodiment of the production method of the nonneural epithelial tissue sheet of the present invention is a method for producing a nonneural epithelial tissue sheet, comprising the following step (a) before the above-mentioned steps (1) - (4):
(a) step a of culturing pluripotent stem cells in the absence of feeder cells and in a medium comprising 1) a TGFβ family signal transduction pathway inhibiting substance and/or a Sonic hedgehog signal transduction pathway activating substance, and 2) a factor for maintaining undifferentiated state.

The step (a), step (1) and step (2) in this method can be performed in the same manner as in step (a), step (1) and step (2) in the above-mentioned production method of the cell mass of the present invention.

### <Step (3)>

In step (3), the step of collecting the nonneural epithelial tissue from the cell mass obtained in the second step is typically performed by detaching and collecting nonneural epithelial tissue existing outside the cell mass by using tweezers and the like under microscopic observation. As a method for collecting the nonneural epithelial tissue in the third step, a freeze-thawing method, preferably a freeze-thawing method using a slow freezing method, can be mentioned and is preferably used. According to the method, a cell mass having nonneural epithelial tissue on the outside and neuron or neural tissue on the inside is subjected to freeze-thawing whereby the nonneural epithelial tissue on the outside is detached from the cell mass without a physical treatment.

### <Step (4)>

In step (4), a nonneural epithelial tissue-derived cell sheet can be formed by subjecting the nonneural epithelial tissue obtained in the third step to a dispersion treatment and culturing the dispersed cells and/or cell aggregate on a flat plane. The dispersion treatment may include mechanical dispersion treatment, cell dispersion solution treatment or cell protecting agent addition treatment, mentioned earlier. These treatments may be performed in combination. For the flat plane culturing of dispersed cells and/or cell aggregate, a method typically performed in the pertinent field is appropriately selected and used.

Another embodiment of the method for producing the nonneural epithelial tissue sheet of the present invention is a production method of a nonneural epithelial tissue sheet including performing the above-mentioned step (a) before the above-mentioned steps (1) - (4).

### 5. Method for performing compound stimulability test

The present invention can provide a method for performing a compound stimulability test using the aforementioned "cell mass containing neural cells or neural tissue and nonneural epithelial tissue" or "nonneural epithelial tissue sheet".

As a method for performing a compound stimulability test, a method for evaluating toxicity or efficacy of a test substance, including a step of bringing a "cell mass containing neural cells or neural tissue and nonneural epithelial tissue" or a "nonneural epithelial tissue sheet" into contact with a test substance, and a step of detecting an influence of the test substance on the cells or tissue can be mentioned.

One embodiment of the method for performing a compound stimulability test of the present invention is a method for performing a compound stimulability test in which toxicity and efficacy of the compound is evaluated by staining with a dye and extraction.

One embodiment of the method for performing a compound stimulability test of the present invention is a method for performing a compound stimulability test including the following steps (A) - (D):
(A) a step of bringing a "cell mass containing neural cells or neural tissue and nonneural epithelial tissue" or a "nonneural epithelial tissue sheet" into contact with a test substance,
(B) a step of staining with a dye the "cell mass containing neural cells or neural tissue and nonneural epithelial tissue" or "nonneural epithelial tissue sheet" contacted with the test substance,
(C) a step of extracting the dye from the stained "cell mass containing neural cells or neural tissue and nonneural epithelial tissue" or "nonneural epithelial tissue sheet",
(D) a step of quantifying the amount of the extracted dye and evaluating the stimulability of the evaluation target compound.

### <Step (A)>

The step of bringing a "cell mass containing neural cells or neural tissue and nonneural epithelial tissue" or a "nonneural epithelial tissue sheet" into contact with a test substance is explained below.

The target to be in contact with a test substance may be a cell mass or a nonneural epithelial tissue sheet. The nonneural epithelial tissue constituting such cell mass or nonneural epithelial tissue sheet preferably forms a tight junction, is more preferably pseudostratified epithelium or stratified epithelium and, further preferably, the nonneural epithelial tissue is cornea. These nonneural epithelial tissues preferably form a basement membrane-like structure. As the nonneural epithelial tissue sheet, any form of a flat plane cell culture dish, and a thin film-like cell culture vessel such as Transwell and the like can be used. Such cell culture dish or cell culture vessel may be coated with an extracellular matrix such as laminin, or a synthetic matrix such as poly-D-lysine to promote cell adhesion.

The test substance to be used in step (A) can be used as it is or used after diluting with a solvent. As the solvent to be used for the dilution, a solvent that does not affect the test results is preferable. For example, physiological saline, PBS, HBSS, DMEM, and a medium for cell culturing can be used. When the test substance is insoluble in the culture medium and good suspension property cannot be achieved, DMSO, ethanol, mineral oil, or the like can be used as a solubilizing solvent as necessary.

The culturing conditions such as culturing temperature, CO₂ concentration and so on in the exposure conditions of the test substance in step (A) can be appropriately determined. The culturing temperature is, for example, about 30°C to about 40°C, preferably about 37°C. The CO₂ concentration is, for example, about 1% to about 10%, preferably about 5%. The exposure period of the test substance in step (A) can also be determined as appropriate. The exposure period is, for example, 30 sec to 2 days, preferably 1 min to 1 day.

To ensure reliability of the experimental system, it is preferable to evaluate known positive control and negative control compounds along with the evaluation of the test substance. As the positive control compound, compounds with GHS classification of 1 or 2 relating to damage or irritation to eyes, for example, sodium dodecyl sulfate, acetic acid, and benzalkonium chloride can be used.

### <step (B)>

A step of staining the "cell mass containing neural cells or neural tissue and nonneural epithelial tissue" or "nonneural epithelial tissue sheet" contacted with the test substance obtained in step (A) with a dye is explained below.

The dye used in step (B) is not limited as long as damage of tight junction caused by the test substance can be evaluated. As the dye, one having low toxicity to cells and low effect on the test results is preferable. One example is fluorescein.

The staining conditions such as concentration of the dye, solvent for dissolving the dye, and staining time in step (B) can be set as appropriate. When fluorescein is used, the concentration of the dye is, for example, 0.0001% to 1%, preferably 0.02%. The solvent for dissolving the dye is, for example, physiological saline, PBS, HBSS, a medium for cell culturing such as DMEM, preferably PBS. In the case of fluorescein, the staining time is, for example, 10 sec to 10 min, preferably 30 seconds. For the purpose of reducing the background, the sample may be washed before and after staining as appropriate under the staining conditions in step (B).

### <Step (C)>

A step of extracting the dye from the stained "cell mass containing neural cells or neural tissue and nonneural epithelial tissue" or "nonneural epithelial tissue sheet" obtained in step (B) is explained below.

The extraction conditions for dye in step (C) such as a solvent for dissolving the dye, and extraction time can be appropriately set. Examples of the solvent for dissolving the dye include physiological saline, PBS, HBSS, a medium for cell culturing such as DMEM, preferably the same as the solvent used for dissolving the dye in step (B). When fluorescein is used, the extraction time is, for example, 10 sec to 24 hr, preferably 10 min. As the extraction conditions in step (C), a tube or dish containing the stained sample may be shaken.

### <Step (D)>

A step of quantifying the amount of the extracted dye obtained in step (C) and evaluating the stimulability of the evaluation target compound is explained below.

The method for quantifying the amount of dye extracted in step (D) is not limited as long as the amount of dye in the solvent can be evaluated. As such quantification method, for example, absorption spectrophotometry and fluorescence spectroscopy can be mentioned, and fluorescence spectroscopy is preferred.

The stimulability of a test substance can be evaluated by comparing measured in step (D), the measurement value of the concentration of the dye extracted from a test substance treatment sample with the measurement values of the positive control treatment group and the negative control treatment group.

### 6. Reagent for evaluation of toxicity and efficacy

The cell mass of the present invention, a cell mass produced by the production method of the present invention, and a nonneural epithelial tissue sheet produced by the present invention may be sensory tissues. Therefore, a reagent for evaluating the toxicity and efficacy of a test substance containing the cell mass of the present invention, a cell mass produced by the production method of the present invention, or a nonneural epithelial tissue sheet produced by the present invention can be provided.

### 7. Therapeutic drug and treatment method of disease

A therapeutic drug for a disease based on a disorder of a sensory organ containing the cell mass of the present invention, a cell mass produced by the production method of the present invention, or a nonneural epithelial tissue sheet produced by the present invention can be provided.

Examples of the therapeutic drug for a disease based on a disorder of a sensory organ include a suspension containing the cell mass of the present invention or a cell mass produced by the production method of the present invention, and a graft containing a nonneural epithelial tissue sheet produced by the present invention.

Examples of the suspension include a liquid obtained by suspending a cell mass in an artificial lacrimal fluid or physiological saline. The suspension may contain nonneural epithelial cells isolated from the cell mass, and may also contain a factor that promotes adhesion of the cells, such as extracellular matrix, and hyaluronic acid.

The graft containing a nonneural epithelial tissue sheet may be a graft in which a nonneural epithelial tissue sheet is formed on a membrane-like carrier such as an amniotic membrane, and a collagen gel membrane.

Furthermore, a method for treating a disease based on a disorder of a sensory organ, including a step of transplanting an effective amount of nonneural epithelial tissue from the cell mass of the present invention, a cell mass produced by the production method of the present invention, or a nonneural epithelial tissue sheet produced by the present invention to a target in need of the transplantation can be provided.

When the nonneural epithelial tissue contained in the cell mass is corneal epithelium tissue, the aforementioned therapeutic drug or treatment method can be used for treating a disease in which corneal epithelial functions such as barrier function and the like are impaired. Examples of the disease include recurrent corneal dystrophy, Stevens-Johnson syndrome, chemical trauma, and corneal epithelium stem cell exhaustion.

The aforementioned disease based on a disorder of a sensory organ may be an animal disease based on a disorder of a sensory organ, or a disease based on a disorder of a sensory organ in a non-human animal, such as vision organ, auditory organ, olfactory organ, sense of taste organ, and skin.

### [Example]

The present invention is explained in more detail in the following by referring to Examples, which are not to be construed as limiting the scope of the present invention. Unless particularly limited, the reagents and materials to be used are commercially available.

### Comparative Example 1: Neural tissue produced from human ES cell

Human ES cells (KhES-1 strain, obtained from Kyoto University) were cultured under feeder-free conditions according to the method described in Scientific Reports, 4, 3594 (2014). As the feeder-free medium, StemFit medium (AK02N, manufactured by Ajinomoto Co., Inc.) was used and, as the feeder-free scaffold, Laminin 511-E8 (manufactured by Nippi, Inc.) was used.

As specific maintenance culture operation, subconfluent human ES cells (KhES-1 strain) were first washed with PBS, subjected to an enzyme treatment using Accumax (manufactured by Innovative Cell Technologies), StemFit medium was added, and the cells were scraped from the surface of the culture dish by using a cell scraper and dispersed into single cells by pipetting. Thereafter, the aforementioned human ES cells dispersed into single cells were seeded in a plastic culture dish coated with Laminin 511-E8, and cultured under feeder-free conditions in StemFit medium in the presence of Y27632 (ROCK inhibiting substance, manufactured by Wako Pure Chemical Industries, Ltd., 10 µM). When a 6-well plate (manufactured by Corning, for cell culture, culture area 9.5 cm²) was used as the aforementioned plastic culture dish, the number of plated cells of the aforementioned human ES cells dispersed into single cells was adjusted to 1.2 x 10⁴. One day after seeding, the entire amount of the medium was changed with StemFit medium free of Y27632. Thereafter, once in 1 - 2 days, the entire amount of the medium was changed with StemFit medium free of Y27632. Thereafter, the cells were cultured until 7 days after seeding when they reached subconfluence (60% of culture area is covered with cells). When the cultured cells were used for differentiation induction, SB-431542 (TGF-β signal transduction pathway inhibiting substance, manufactured by Wako Pure Chemical Industries, Ltd., 5 µM) and SAG (Shh signal pathway activating substance, manufactured by Enzo Life Sciences, 300 nM) were added at 6 days after seeding and simultaneously with the medium change with Stemfit medium.

The thus-prepared subconfluent human ES cells were washed with PBS, subjected to an enzyme treatment using Accumax, a serum-free medium for differentiation induction was added, and the cells were scraped from the surface of the culture dish by using a cell scraper and dispersed into single cells by pipetting.

Thereafter, the aforementioned human ES cells dispersed into single cells were suspended in 100 µl of a serum-free medium at 1 x 10⁴ cells per well of a non-cell-adhesive 96-well culture plate (PrimeSurface 96V-bottom plate, manufactured by SUMITOMO BAKELITE), and cultured in suspension under the conditions of 37°C, 5% CO₂. As the serum-free medium (gfCDM+KSR) therefor, a serum-free medium which is a 1:1 mixture of F-12+Glutamax medium (manufactured by Thermo Fisher Scientific) and IMDM+Glutamax medium (manufactured by Thermo Fisher Scientific) supplemented with 5% Knockout Serum Replacement (manufactured by Thermo Fisher Scientific), 450 µM 1-mono thioglycerol (manufactured by Wako Pure Chemical Industries, Ltd.), 1x Chemically defined lipid concentrate (manufactured by Thermo Fisher Scientific), 50 unit/ml penicillin-50 µg/ml streptomycin (manufactured by Nacalai Tesque) was used. At the time of the start of suspension culturing (day 0 after the start of suspension culturing, start of step 1), Y27632 (final concentration 20 µM), IWP-2 (Wnt signal transduction pathway inhibiting substance, manufactured by Tocris Bioscience, 2 µM), and SB-431542 (TGF-β signal transduction pathway inhibiting substance, manufactured by Wako Pure Chemical Industries, Ltd., 1 µM) were added to the aforementioned serum-free medium. Thereafter, a serum-free medium not containing Y27632 and containing IWP-2 and SB-431542 was added at 100 µl per well at 3 days after the start of suspension culturing. Thereafter, a half amount of the medium was changed with a serum-free medium not containing Y27632 and containing IWP-2 and SB-431542 (also abbreviated as SB431 in Fig. 1) was added at 6, 10, 13, 17, 21, 24 days after the start of suspension culturing. Cell masses were collected in a dish 28 days after the start of suspension culturing, and subjected to bright field observation under an inverted microscope (manufactured by KEYENCE CORPORATION, BIOREVO) (Figs. 1A-B). The scale bar at the lower right of Fig. 1A shows 1000 µm, and the scale bar at the lower right of Fig. 1B shows 200 µm. As a result, cell masses were formed from human pluripotent stem cells by the above-mentioned differentiation induction method.

The aforementioned cell masses at 28 days after the start of suspension culturing were each fixed with 4% para-formaldehyde at room temperature for 15 min, immersed in 20% sucrose/PBS at 4°C overnight as a cryoprotection treatment, and cryosections were prepared. The cryosections were subjected to fluorescence immunostaining with an antibody against RLDH3 that is expressed in nerve and retinal tissues (manufactured by Sigma Aldrich, rabbit), an anti-ChxlO antibody (manufactured by Santa Cruz Biotechnology, goat), an anti-Rx antibody (manufactured by Takara Bio Inc., guinea pig), an anti-Bf1 antibody (manufactured by Takara Bio Inc., rabbit) which is a cerebral cortex marker, an anti-Pax6 antibody (manufactured by Covance, rabbit) which is a cornea and central nervous system marker, an anti-pan-cytokeratin (Pan CK) antibody (manufactured by Sigma Aldrich, mouse) which is a cornea and nonneural tissue marker, an anti-βIII tubulin (Tuj1) antibody (manufactured by Sigma Aldrich Ltd., mouse) which is a neuron marker, or an anti-N-Cadherin antibody (manufactured by BD Bioscience, mouse) which is a neuroepithelial marker. Multiple staining was performed using, as fluorescence-labeled secondary antibodies, Alexa488-labeled donkey anti-rabbit antibody (manufactured by Thermo Fisher Scientific), CF555-labeled donkey anti-mouse antibody (manufactured by Biotium), CF555-labeled donkey anti-goat antibody, CF543-labeled donkey anti-guinea pig antibody, and Alexa647-labeled donkey anti-mouse antibody. Hoechst33342 (manufactured by Sigma Aldrich) was used for comparison staining of nucleus.

Upright fluorescence microscope Axio Imager M2 and the attached software, Axio Vision (manufactured by Carl Zeiss), were used for observation and obtainment of images of the stained sections. The scale bar at the lower right of Fig. 1C shows 200 µm. Fig. 1F is a comparison stained image of the nucleus for Figs. 1C, D, E, Fig. 1J is a comparison stained image of the nucleus for Figs. 1G, H, I, and Fig. 1M is a comparison stained image of the nucleus for Figs. 1K and L.

As a result, since the cell masses at 28 days after the start of suspension culturing which were induced by the above-mentioned differentiation induction method were negative for RLDH3, Chx10, Rx expressed in retina (Fig. 1C, D, G), positive for Bf1, Pax6 expressed in cerebrum and central nervous system (Figs. 1K, H), and positive for Tuj1, N-Cadherin which are neuron markers (Figs. 1I, L), it was found that they are cell masses formed from the cells in the central nervous system. Since pan-cytokeratin positive tissue cannot be confirmed, it was found that nonneural epithelial tissue was not contained (Fig. 1E).

### Example 1: cell mass containing neural tissue and nonneural epithelial tissue produced from human ES cells

Human ES cells (KhES-1 strain, obtained from Kyoto University) were cultured under feeder-free conditions according to the method described in Scientific Reports, 4, 3594 (2014). As the feeder-free medium, StemFit medium was used and, as the feeder-free scaffold, Laminin 511-E8 was used. A specific maintenance culturing operation was performed in the same manner as in Comparative Example 1. Thereafter, suspension culturing in a 96-well plate under conditions similar to those in Comparative Example 1 was started. Thereafter, a serum-free medium not containing Y27632 and containing IWP-2, SB-431542, BMP4 was added at 100 µl per well on day 2 from the start of suspension culturing. BMP4 was added at 3 nM to the medium such that the final concentration thereof in the well was 1.5 nM. Thereafter, a half amount of the medium was changed with a serum-free medium not containing Y27632 or BMP and containing IWP-2 and SB-431542 was added on days 6, 10, 13, 17, 21, 24 from the start of suspension culturing. Cell masses were collected in a dish on day 28 from the start of suspension culturing, and subjected to bright field observation under an inverted microscope (manufactured by KEYENCE CORPORATION, BIOREVO) (Figs. 2A-B). The scale bar at the lower right of Fig. 2A shows 1000 µm, and the scale bar at the lower right of Fig. 2B shows 200 µm. As a result, cell masses with a diameter of about 1 mm and containing neural tissue and nonneural epithelial tissue were formed from human ES cells by the above-mentioned differentiation induction method. Furthermore, it was found that the nonneural epithelial tissue on the outside is rapidly swollen from day 21 to day 28 of suspension culturing and a space is formed between the tissue and the neural tissue in the inside.

The aforementioned cell masses on day 28 from the start of suspension culturing were each fixed with 4% para-formaldehyde at room temperature for 15 min, immersed in 20% sucrose/PBS at 4°C overnight, and subjected to a cryoprotection treatment, and cryosections were prepared. The cryosections were subjected to fluorescence immunostaining with an anti-Chx10 antibody, an anti-RLDH3 antibody, an anti-CD56/NCAM antibody (manufactured by BioLegend, mouse), an anti-N-Cadherin antibody which are nerve and retinal tissue markers, an anti-CD326/EpCAM antibody (manufactured by R&D Systems, goat), an anti-Sixl antibody (manufactured by Sigma Aldrich, rabbit), an anti-p63 antibody (manufactured by Santa Cruz Biotechnology, mouse), an anti-PDGFRβ antibody (manufactured by R&D Systems, goat), an anti-cytokeratin 18 antibody (manufactured by Sigma Aldrich, mouse), an anti-cytokeratin 19 antibody (manufactured by Thermo Fisher Scientific, mouse), an anti-pan-cytokeratin antibody which are nonneural epithelial tissue and cornea markers, an anti-Laminin antibody (manufactured by KYOWA PHARMA CHEMICAL CO.,LTD., mouse) which is a basement membrane marker, an anti-βIII tubulin (Tuj1) antibody which is a neuron marker, an anti-C-Maf antibody (manufactured by R&D Systems, mouse), an anti-Proxl antibody (manufactured by R&D Systems, goat), an anti-L-Maf antibody (manufactured by abcam, rabbit), an anti-Crystallin αA antibody (manufactured by R&D Systems, goat) which is a crystallin lens marker, an anti-Sox1 antibody (manufactured by R&D Systems, goat) which is a crystallin lens and central nervous system marker, an anti-Emx2 antibody (manufactured by R&D Systems, sheep) which is a placode marker, and an anti-acetylated tubulin antibody (manufactured by Santa Cruz Biotechnology, mouse) which is a stabilized microtubule marker. Multiple staining was performed using, as fluorescence-labeled secondary antibodies, Alexa488-labeled donkey anti-rabbit antibody, CF555-labeled donkey anti-mouse antibody, CF555-labeled donkey anti-goat antibody, CF543-labeled donkey anti-sheep antibody, Alexa647-labeled donkey anti-mouse antibody, and Alexa647-labeld donkey anti-goat antibody. Hoechst 33342 (manufactured by Sigma Aldrich) was used for comparison staining of nucleus. Fig. 2D is a comparison stained image of the nucleus for C, Fig. 2F is that for E, Fig. 2H is that for G, Fig. 2J is that for I, Fig. 2N is that for K, L and M, Fig. 2P is that for O, Fig. 2R is that for Q, Fig. 2T is that for S, Fig. 2V is that for U, Fig. 2Y is that for W and X, Fig. 2AB is that for Z and AA, Fig. 2AE is that for AC and AD, Fig. 2AH is that for AF and AG, Fig. 2AK is that for AI and AJ, Fig. 2AN is that for AL and AM, Fig. 2AP is that for AO, Fig. 2AR is that for AQ, Fig. 2AV is that for AS, AT and AU. Upright fluorescence microscope Axio Imager M2 and the attached software, Axio Vision, were used for observation and obtainment of images of the stained sections. The scale bar at the lower right of Fig. 2C, Fig. 2S shows 200 µm, and the scale bar at the lower right of Fig. 2W, AO shows 100 µm.

As a result, it was found that the inside of the cell masses on day 28 from the start of suspension culturing which were induced by the above-mentioned differentiation induction method was Chx10, RLDH3, NCAM, N-Cadherin positive epithelial tissue of neural retina (Figs. 2C-J), and the outside was EpCAM, Six1, p63, PDGFRβ, cytokeratin 18, 19, pan-cytokeratin positive nonneural epithelial tissue of cornea and eye surface ectoderm (Figs. 2K-V). In addition, a part of the nonneural epithelial tissue was thickened to a thickness of about 100 µm, and the thickened part was C-Maf, L-Maf, Sox1, Prox1, Emx2, Pax6, N-Cadherin, Crystalline αA positive and Tuj1 negative. It was found that lens placode was formed in the cell masses by the above-mentioned production method, lens vesicle was invaginated, pan-cytokeratin, EpCAM, Six1 positive cornea tissue was formed to cover the surface of the invaginated crystallin lens, and Laminin-positive basement membrane tissue was formed on the side in contact with the retinal tissue near the center of the formed crystallin lens. In addition, since the Laminin-positive basement membrane tissue was formed only on one surface of the epithelial tissue, it was found that the formed cornea tissue has epithelial cell polarity. Furthermore, since the nuclei of the formed cornea tissue are layered, it was found that the formed cornea tissue was pseudostratified epithelium or stratified epithelium (Figs. 2W-AR). In addition, it was found that pan-cytokeratin-positive, non-epithelial mesenchymal cells are present between the inner retinal tissue positive for RLDH3 and Chx10 and the outer nonneural epithelial tissue positive for pan-cytokeratin (Figs. 2AS-AV). A schematic drawing of the cell mass formed by the above-mentioned production method is shown in Fig. 2AW.

### Example 2: Measurement of space between neural tissue and nonneural epithelial tissue in cell mass produced from human ES cells

The aforementioned cell masses on day 28 from the start of suspension culturing and obtained in the aforementioned Example 1 were subjected to fluorescence double staining according to the method described in Example 1 and using an anti-N-Cadherin antibody which is a nerve and retinal epithelial tissue marker and an anti-CD326/EpCAM antibody which is a nonneural epithelial tissue and corneal marker, and analyzed using Axio Vision which is a software attached to a fluorescence microscope (Figs. 3A-D). The scale bar at the lower right of Fig. 3A, Fig. 3D shows 200 µm. As a result, the space formed between the N-Cadherin positive neural tissue in the inside of the cell masses at 28 days of culture and EpCAM positive nonneural epithelial tissue on the outside thereof is 33.37 µm at minimum and 118.34 µm at maximum, and it was found that a space of not less than 30 µm was formed between neuroepithelium and nonneural epithelium on almost the entire circumference of the cell masses (Figs. 3A-D). The above-mentioned space is free from nucleus and is acellular; however, distribution of non-epithelial cells could be confirmed in a part thereof.

### Example 3: Long-term culturing and maturation of cell mass containing neural tissue and nonneural epithelial tissue and produced from human ES cells

Human ES cells (KhES-1 strain, obtained from Kyoto University) were cultured under feeder-free conditions according to the method described in Scientific Reports, 4, 3594 (2014). As the feeder-free medium, StemFit medium was used and, as the feeder-free scaffold, Laminin 511-E8 was used. A specific maintenance culturing operation was performed in the same manner as in Comparative Example 1. Thereafter, suspension culturing was performed using a 96-well plate under the same conditions as in Example 1. On day 28 from the start of suspension culturing, cell masses were placed in a 10 cm dish for suspension culture (manufactured by SUMITOMO BAKELITE CO., LTD.) at 48 cell masses per one dish, and cultured in suspension in a serum-free medium up to day 90 of culturing. As the serum-free medium (gfCDM+KSR) therefor, a serum-free medium which is a 1:1 mixture of F-12+Glutamax medium (manufactured by Thermo Fisher Scientific) and IMDM+Glutamax medium (manufactured by Thermo Fisher Scientific) supplemented with 10% Knockout Serum Replacement (manufactured by Thermo Fisher Scientific), 450 µM 1-mono thioglycerol (manufactured by Wako Pure Chemical Industries, Ltd.), 1x Chemically defined lipid concentrate (manufactured by Thermo Fisher Scientific), 50 unit/ml penicillin-50 µg/ml streptomycin (manufactured by Nacalai Tesque) was used at 15 ml per one dish, and a half amount of the medium was changed every 3 to 4 days.

The aforementioned cell masses on day 90 from the start of suspension culturing were subjected to bright field observation under an inverted microscope (manufactured by KEYENCE CORPORATION, BIOREVO) (Figs. 4A-C). The scale bar at the lower right of Fig. 4A shows 1000 µm, and the scale bar at the lower right of Figs. 4B, 4C shows 200 µm. As a result of observation, the tissue outside the cell mass on day 90 of culturing autonomously maintained a spherical structure in the culture medium (Fig. 4A). The hollow tissue on the outside was formed from epithelial tissue in which cells were closely adhered to each other (Fig. 4C). After observation, the cell masses were fixed with 4% para-formaldehyde at room temperature for 15 min, immersed in 20% sucrose/PBS at 4°C overnight, and subjected to a cryoprotection treatment, and cryosections were prepared. The cryosections were subjected to fluorescence immunostaining with an anti-cytokeratin 12 antibody (manufactured by Santa Cruz Biotechnology, goat) which is a mature corneal epithelium marker, an anti-cytokeratin 5 antibody (manufactured by Spring Biosciences, rabbit) and an anti-Mucin4 antibody (manufactured by R&D Systems, goat) which are cornea epithelial cell markers, an anti-Pax6 antibody which is a cornea and central nervous system cell marker, and an anti-Laminin antibody which is a basement membrane marker under the conditions similar to those in Example 1. The scale bar at the lower right of Figs. 4D, 4H, 4L shows 200 µm.

As a result, in the cell mass at day 90 of culturing, the nonneural epithelial tissue on the outside was elongated, and a space of not less than 200 µm was formed. It was found that the above-mentioned nonneural epithelial tissue is cytokeratin 12, cytokeratin 5, Mucci4-positive corneal epithelium tissue and has basement membrane tissue constituted of Laminin and the like. In addition, it was found that Mucin4 expressed on the apical side of corneal epithelial cell membrane surface is localized on the outer surface of nonneural epithelial tissue, and the formed nonneural epithelial tissue has epithelial cell polarity. The inside tissue was Pax6-positive retinal or central nervous system tissue. From the above-mentioned results, it was found that matured corneal epithelium tissue was obtained from the cell masses by suspension culturing (Figs. 4D-N).

### Example 4: Consideration of timing of addition of BMP4 in production of cell mass containing neural tissue and nonneural epithelial tissue from human ES cells (1)

Human ES cells (KhES-1 strain, obtained from Kyoto University) were cultured under feeder-free conditions according to the method described in Scientific Reports, 4, 3594 (2014). As the feeder-free medium, StemFit medium was used and, as the feeder-free scaffold, Laminin 511-E8 was used.

Specific maintenance culturing operation was performed in the same manner as in Comparative Example 1. Thereafter, suspension culturing in a 96-well plate under conditions similar to those in Comparative Example 1 was started. The condition without addition of a BMP signal transduction pathway activating substance during the suspension culturing period was taken as the control (-BMP4 condition, Fig. 5A). In the condition with the addition of a BMP signal transduction pathway activating substance simultaneously with the start of the suspension culturing (Day0+BMP4 condition, Fig. 5B), human recombinant BMP4 (manufactured by R & D Systems, 1.5 nM, start of step 2) was added. Thereafter, in the condition with the addition of a BMP signal transduction pathway activating substance on days 1, 2, 3 from the start of suspension culturing (Day1 - 3+BMP4 condition, Figs. 5C-E), a serum-free medium not containing Y27632 and containing IWP-2, SB-431542, BMP4 was added at 100 µl per well. BMP4 was added at 3 nM to the medium such that the final concentration thereof in the well was 1.5 nM. Only in the Day0+BMP4 condition, a serum-free medium not containing Y27632 and containing IWP-2, SB-431542, BMP4 was added at 100 µl per well on day 3 from the start of suspension culturing. In the condition with the addition of a BMP signal transduction pathway activating substance on day 6 from the start of differentiation induction (Day6+BMP4 condition, Fig. 5F), a serum-free medium not containing Y27632 or BMP4 and containing IWP-2 and SB-431542 was added at 100 µl per well on day 3 from the start of differentiation induction, and a half amount of the medium was changed on day 6 with a serum-free medium not containing Y27632 and containing IWP-2, SB-431542, BMP4. Under other conditions, a half amount of the medium was changed with a serum-free medium not containing Y27632 or BMP and containing IWP-2 and SB-431542 on day 6 from the start of suspension culturing. On day 10 from the start of suspension culturing, bright field observation was performed using an inverted microscope (manufactured by KEYENCE CORPORATION, BIOREVO) (Figs. 5A-B).

As a result, under the condition without addition of BMP4, embryoid body with a smooth surface was formed, and a nonneural epithelium-like structure could not be confirmed on the embryoid body surface (Fig. 5A). In the condition with the addition of BMP4 simultaneously with the start of differentiation induction (Day0+BMP4), formation of embryoid body was markedly inhibited (Fig. 5B). In the condition with the addition of BMP4 on day 1 and day 2 from the start of differentiation induction (Day1, 2+BMP4), cell masses containing neural tissue and nonneural epithelial tissue and having a two-layer structure in which a center part with a neuroepithelium-like structure is covered with a nonneural epithelium-like layer were formed (Figs. 5C, D). By a comparison of the both conditions, addition of BMP4 on day 2 resulted in the formation of embryoid body which is about 1.3 times larger in the diameter and about 2.2 times greater in the volume (Fig. 5D) than the embryoid body obtained under the condition with the addition of BMP4 on day 1 (Fig. 5C). In the condition with the addition of BMP4 on day 3 and day 6 from the start of differentiation induction, the efficiency of formation of nonneural epithelium-like tissue on the outside was lower than that of the condition with the addition of BMP4 on day 2. From the above-mentioned results, it was found that addition of a BMP signal transduction pathway activating substance within 72 hr from the start of suspension culturing is effective for the formation of a cell mass containing neural tissue and nonneural epithelial tissue from human pluripotent stem cells.

### Example 5: Consideration of timing of addition of BMP4 in production of cell mass containing neural tissue and nonneural epithelial tissue from human ES cells (2)

Human ES cells (KhES-1 strain, obtained from Kyoto University) were cultured under feeder-free conditions according to the method described in Scientific Reports, 4, 3594 (2014). As the feeder-free medium, StemFit medium was used and, as the feeder-free scaffold, Laminin 511-E8 was used.

Specific maintenance culturing operation was performed in the same manner as in Comparative Example 1. Thereafter, suspension culturing in a 96-well plate under conditions similar to those in Comparative Example 1 was performed. Bright field observation of aggregates was performed using an inverted microscope on day 2 and day 3 from the start of suspension culturing. The scale bar at the lower right of Fig. 6A shows 200 µm. As a result of observation, the aggregate on day 2 from the start of suspension culturing which is suitable for the addition of BMP4 showed concaves and convexes on the surface, and had a distorted shape (Fig. 6A). On the other hand, the aggregate on day 3 from the start of suspension culturing showed a decrease in the concaves and convexes found on day 2, and had a shape close to a sphere (Fig. 6B).

The aforementioned, the aggregates on day 2 and day 3 from the start of suspension culturing were fixed by the method described in Example 1 and cryosections were prepared. The cryosections were subjected to fluorescence immunostaining using an anti-N-Cadherin (NCad) antibody which is a neuroepithelial marker and an anti-ZO-1 antibody (manufactured by Thermo Fisher Scientific, rabbit) which is a tight junction marker. The fluorescence-labeled secondary antibody and comparison staining of nucleus used were those described in Example 1. The scale bar at the lower right of Fig. 6C shows 100 µm. As a result, in the aggregate on day 2 from the start of suspension culturing, a part of the cells closest to the surface layer of the aggregate was ZO-1 positive and formed a tight junction (Fig. 6C). On the other hand, in the aggregate on day 3 from the start of suspension culturing, ZO-1 positive cells were taken up inside the aggregate and localization was not seen on the outermost layer (Fig. 6E). In addition, in the aggregate on day 3 from the start of suspension culturing, N-Cadherin was strongly expressed, and the two-layer structure of the region where the cells in the outer layer are more closely adhered to each other and the region where the cells inside the aggregate are sparse could be more clearly confirmed (Figs. 6I, J). From the above-mentioned results, it was found that detection of tight junction of the cells in the outermost layer of aggregates can be used as a method for determining the timing of addition of BMP4 in the process of producing a cell mass containing neural tissue and nonneural epithelial tissue.

### Example 6: Consideration of concentration of BMP4 in production of cell mass containing neural tissue and nonneural epithelial tissue from human ES cells

Human ES cells (KhES-1 strain, obtained from Kyoto University) were cultured under feeder-free conditions according to the method described in Scientific Reports, 4, 3594 (2014). As the feeder-free medium, StemFit medium was used and, as the feeder-free scaffold, Laminin 511-E8 was used. Specific maintenance culturing operation was performed in the same manner as in Comparative Example 1.

The thus-prepared subconfluent human ES cells were washed with PBS, subjected to an enzyme treatment using Accumax, a serum-free medium for differentiation induction was added, and the cells were scraped from the surface of the culture dish by using a cell scraper and dispersed into single cells by pipetting. Thereafter, the aforementioned human ES cells dispersed into single cells were suspended in 100 µl of a serum-free medium at 1 x 10⁴ cells per well of a non-cell-adhesive 96-well culture plate (PrimeSurface 96V-bottom plate, manufactured by SUMITOMO BAKELITE), and cultured in suspension under the conditions of 37°C, 5% CO₂. As the serum-free medium (gfCDM+KSR) therefor, a serum-free medium which is a 1:1 mixture of F-12+Glutamax medium (manufactured by Thermo Fisher Scientific) and IMDM+Glutamax medium (manufactured by Thermo Fisher Scientific) supplemented with 5% Knockout Serum Replacement (manufactured by Thermo Fisher Scientific), 450 µM 1-mono thioglycerol (manufactured by Wako Pure Chemical Industries, Ltd.), 1x Chemically defined lipid concentrate (manufactured by Thermo Fisher Scientific), 50 u/ml penicillin-50 µg/ml streptomycin (manufactured by Nacalai Tesque) was used. At the time of the start of suspension culturing (day 0 from the start of suspension culturing, start of step 1), Y27632 (final concentration 20 µM), IWP-2 (2 µM), and SB-431542 (1 µM) were added to the aforementioned serum-free medium. Thereafter, a serum-free medium not containing Y27632 and containing IWP-2, SB-431542 and BMP4 was added at 100 µl per well on day 2 from the start of suspension culturing. In this case, the amounts of BMP4 to be added were 8 conditions of 0.1 nM, 0.25 nM, 0.5 nM, 0.75 nM, 1 nM, 1.5 nM, 5 nM and no addition control. BMP4 was added at twice the concentration set for the medium so that the final concentration set for the well could be achieved. Thereafter, a half amount of the medium was changed with a serum-free medium not containing Y27632 or BMP and containing IWP-2 and SB-431542 on day 6 from the start of suspension culturing. Bright field observation was performed under an inverted microscope (manufactured by KEYENCE CORPORATION, BIOREVO) on day 10 from the start of suspension culturing (Figs.7A-H). The scale bar at the lower right of Fig. 7A shows 200 µm.

As a result, under the conditions without addition of BMP4, embryoid body with a smooth surface was formed, and a nonneural epithelium-like structure could not be confirmed on the embryoid body surface (Fig. 7A). On the other hand, under the conditions with the addition of BMP4, a neuroepithelium-like cell mass was present in the inside at any concentration of from 0.1 nM to 5 nM, and a cell mass having nonneural epithelial tissue on the surface was formed (Figs. 7B-H). From the above-mentioned results, it was found that when BMP4 is added on day 2 from the start of suspension culturing, nonneural epithelial tissue can be formed on the surface of cell mass as long as the concentration is not less than 0.1 nM.

### Example 7: Effect of each Wnt signal transduction pathway inhibiting substance on production of cell mass containing neural tissue and nonneural epithelial tissue from human ES cells

Human ES cells (KhES-1 strain) were cultured under feeder-free conditions according to the method described in Scientific Reports, 4, 3594 (2014). As the feeder-free medium, StemFit medium was used and, as the feeder-free scaffold, Laminin 511-E8 was used. Specific maintenance culturing operation was performed in the same manner as in Example 1.

The thus-prepared subconfluent human ES cells were washed with PBS, subjected to an enzyme treatment using Accumax, a mukessei medium medium for differentiation induction was added, and the cells were scraped from the surface of the culture dish by using a cell scraper and dispersed into single cells by pipetting. Thereafter, the aforementioned human ES cells dispersed into single cells were suspended in 100 µl of a serum-free medium at 1.2 x 10⁴ cells per well of a non-cell-adhesive 96-well culture plate (PrimeSurface 96V-bottom plate, manufactured by SUMITOMO BAKELITE), and cultured in suspension under the conditions of 37°C, 5% CO₂. As the serum-free medium (gfCDM+KSR) therefor, a serum-free medium which is a 1:1 mixture of F-12+Glutamax medium and IMDM+Glutamax medium supplemented with 5% Knockout Serum Replacement, 450 µM 1-mono thioglycerol, 1x Chemically defined lipid concentrate, 50 u/ml penicillin-50 µg/ml streptomycin was used. At the time of the start of suspension culturing (day 0 from the start of suspension culturing, start of step 1), Y27632 (final concentration 20 µM) and SB-431542 (1 µM) were added to the aforementioned serum-free medium. As the Wnt signal transduction pathway inhibiting substance, IWP-2 (manufactured by Tocris Bioscience, 2 or 5 µM), C-59 (manufactured by Cayman Chemicals, 2 µM), IWP-L6 (manufactured by AdooQ bioscience, 1 or 10 µM), LGK974 (manufactured by Cayman Chemicals, 1 or 5 µM), KY 02111 (manufactured by Cayman Chemicals, 1 or 5 µM), or XAV939 (manufactured by Cayman Chemicals, 1 µM) was added at the start of the suspension culturing, and the condition with the addition of DMSO alone as a control of no addition of a Wnt signal transduction pathway inhibiting substance was performed. Thereafter, a serum-free medium not containing Y27632 and containing each Wnt signal transduction pathway inhibiting substance, SB-431542 and BMP4 was added at 100 µl per well on day 2 from the start of suspension culturing. BMP4 was added at 3 nM to the medium so that the final concentration in the well could be 1.5 nM. A half amount of the medium was changed with a serum-free medium not containing Y27632 or BMP and containing each Wnt signal transduction pathway inhibiting substance and SB-431542 on day 6 from the start of suspension culturing. Bright field observation was performed under an inverted microscope (manufactured by KEYENCE CORPORATION, BIOREVO) on day 10 from the start of suspension culturing (Figs. 8A-K). The scale bar at the lower right of Fig. 8A shows 200 µm.

As a result, in the condition without the addition of a Wnt signal transduction pathway inhibiting substance, nonneural epithelial tissue was not formed on the surface of embryoid body even when BMP4 was added on day 2 from the start of suspension culturing (Fig. 8A). On the other hand, under respective conditions with the addition of IWP-2, C-59, IWP-L6, LGK974, KY02111, XAV939 from the time point of the start of suspension culturing, a neuroepithelium-like cell mass was present in the inside, and a cell mass having nonneural epithelial tissue on the surface was formed (Figs. 8B-K). From the above-mentioned results, it was found that the addition of a Wnt signal transduction pathway inhibiting substance is useful for the formation of a cell mass having nonneural epithelial tissue by the addition of BMP4.

### Example 8: Effect of compound treatment before differentiation induction in production of cell mass containing neural tissue and nonneural epithelial tissue from human ES cells

Human ES cells (KhES-1 strain) were cultured under feeder-free conditions according to the method described in Scientific Reports, 4, 3594 (2014). As the feeder-free medium, StemFit medium was used and, as the feeder-free scaffold, Laminin 511-E8 was used. Specific maintenance culturing operation was performed in the same manner as in Example 1. Cells under 4 conditions were prepared including conditions in which, when the cultured cells are used for differentiation induction, nothing is simultaneously added with medium change with Stemfit medium (Control), conditions in which 300 nM SAG alone is added (+300 nM SAG), conditions in which 5 µM SB-431542 alone is added (+5 µM SB-431542), and conditions in which SAG and SB431542 are simultaneously added (+SAG/SB) at 6 days after seeding.

The next day of the above-mentioned treatment, human ES cells under 4 conditions that reached subconfluence were each subjected to differentiation induction by suspension culturing into a cell mass containing neural tissue and nonneural epithelial tissue according to the method described in Example 1. Thereafter, on day 15 of differentiation induction, bright field observation was performed using an inverted microscope (manufactured by KEYENCE CORPORATION, BIOREVO). The cell masses formed after each pre-treatment were evaluated in three stages of cell masses with near spherical form in which not less than 80% of the whole circumference is coated with nonneural epithelium (Grade 1, e.g.=Fig. 9A); cell masses in which 80% to 40% of the whole circumference is coated with nonneural epithelium, or with a distorted shape (Grade 2, e.g.=Fig. 9B); and cell masses in which the proportion of nonneural epithelium on the cell mass surface is not more than 40% (Grade 3, e.g.=Fig. 9C).

As a result, cultured cells are used for differentiation induction, 32 cell masses under the condition with no addition simultaneously with the medium change of Stemfit medium 6 days after seeding (Control) contained 6 Grade 1 cell masses, 18 Grade 2 cell masses, and 8 Grade 3 cell masses. The 32 cell masses under the condition with addition of 300 nM SAG alone (+300 nM SAG) contained 10 Grade 1 cell masses, 18 Grade 2 cell masses, and 4 Grade 3 cell masses. The 32 cell masses under the condition with addition of 5 µM SB-431542 alone (+5 µM SB-431542) contained 28 Grade 1 cell masses, 4 Grade 2 cell masses, and 0 Grade 3 cell mass. The 32 cell masses under the condition with simultaneous addition of SAG and SB431542 (+SAG/SB) contained 30 Grade 1 cell masses, 2 Grade 2 cell masses, and 0 Grade 3 cell mass. The evaluation results are shown in Fig. 9D as a graph. From the above-mentioned results, it was shown that the proportion of Grade 1 cell masses with near spherical form in which not less than 80% of the whole circumference is coated with nonneural epithelium as compared to Control with no treatment is drastically improved by performing a pre-treatment with 5 µM SB-431542 or a pre-treatment by simultaneous addition of SAG and SB431542 when the cultured cells are used for differentiation induction (Fig. 9D).

### Example 9: Cell mass containing neural tissue and nonneural epithelial tissue and produced from human iPS cells

Human iPS cells (201B7 strain, obtained from iPS Academia Japan, Inc.) were cultured under feeder-free conditions according to the method described in Scientific Reports, 4, 3594 (2014). As the feeder-free medium, StemFit medium was used and, as the feeder-free scaffold, Laminin 511-E8 was used. A specific maintenance culturing operation was performed in the same manner as in Comparative Example 1. Thereafter, suspension culturing was performed using a 96-well plate under the same conditions as in Example 1. A serum-free medium not containing Y27632 and containing IWP-2, SB-431542 and BMP4 was added at 100 µl per well on day 2 from the start of suspension culturing. BMP4 was added at 3 nM to the medium so that the final concentration in the well could be 1.5 nM. A half amount of the medium was changed with a serum-free medium not containing Y27632 or BMP and containing IWP-2 and SB-431542 on day 6 from the start of suspension culturing. Bright field observation was performed under an inverted microscope (manufactured by KEYENCE CORPORATION, BIOREVO) on day 10 and day 28 from the start of suspension culturing (Figs. 10A-C). The scale bar at the lower right of Figs. 10A and C shows 200 µm, and the scale bar at the lower right of Fig. 10B shows 100 µm. As a result, it was found that a cell mass in which nonneural epithelial tissue surrounds neural tissue of the center part from the human iPS cell line 201B7 was formed as in the case of production from human ES cell line KhES-1 in Example 1.

Cryosections were prepared by the method described in Example 1 from the cell masses prepared from the aforementioned human iPS cell line 201B7 and on day 28 from the start of suspension culturing and fluorescence immunostaining was performed using an anti-ChxlO antibody, an anti-RLDH3 antibody, an anti-CD56/NCAM antibody, an anti-N-Cadherin antibody, an anti-Pax6 antibody or an anti-Rx antibody which is a nerve and retinal tissue marker, an anti-CD326/EpCAM antibody, an anti-E-Cadherin antibody (manufactured by R&D Systems, goat), an anti-Six1 antibody, an anti-p63 antibody (manufactured by Santa Cruz Biotechnology, rabbit), or an anti-pan-cytokeratin antibody which is a nonneural epithelial tissue and corneal marker, or an anti-β III tubulin (Tuj1) antibody which is a neuron marker. Fluorescence-labeled secondary antibody and conditions of microscopic observation were as described in the method of Example 1. Fig. 10G is a comparison stained image of the nucleus for Figs. 10D-F, Fig. 10K is that for Figs. 10H-J, Fig. 10O is that for Figs. 10L-N, and Fig. 10S is that for Figs. 10P-R.

As a result, it was found that cell masses in which the inside is Chx10, RLDH3, NCAM, N-Cadherin, Pax6, Rx, Tuj1 positive neural retinal tissue, and the outside is EpCAM, E-Cadherin, Six1, p63, pan-cytokeratin positive nonneural epithelial tissue were formed from iPS cell line 201B7 by the above-mentioned production method, as in the case of production from human ES cell line KhES-1 in Example 1 (Figs. 10D-S).

### Example 10: Production Example of corneal epithelium sheet from human ES cells

Cell masses were prepared by the method described in Example 3 from human ES cell line KhES-1. Cell masses were collected by micro pipetting on day 28 from the start of suspension culturing, placed in a dish for suspension culture (manufactured by SUMITOMO BAKELITE CO., LTD.), and further cultured in suspension under the conditions of 37°C, 5% CO₂ for 6 days. As the serum-free medium (gfCDM+KSR) therefor, a serum-free medium which is a 1:1 mixture of F-12+Glutamax medium (manufactured by Thermo Fisher Scientific) and IMDM+Glutamax medium (manufactured by Thermo Fisher Scientific) supplemented with 10% Knockout Serum Replacement (manufactured by Thermo Fisher Scientific), 450 µM 1-mono thioglycerol (manufactured by Wako Pure Chemical Industries, Ltd.), 1xChemically defined lipid concentrate (manufactured by Thermo Fisher Scientific), 50 unit/ml penicillin-50 µg/ml streptomycin (manufactured by Nacalai Tesque) was used. To isolate corneal epithelial cells, the neural tissue in the inside was removed from the embryoid body on day 34 from the start of suspension culturing by using No.5 precision tweezers under a stereoscopic microscope and only the corneal epithelium tissue on the outside was recovered. Bright field observation under an inverted microscope was performed before (Fig. 11A) and after (Fig. 11B) recovery of corneal epithelial tissue. The scale bar at the lower right of Fig. 11A shows 200 µm. As a result of observation, it was found that corneal epithelium tissue present on the outside of the cell masses could be efficiently isolated (Fig. 11B). Thereafter, the recovered tissue was washed with PBS, Accumax+10 µM Y-27632 was added, and the mixture was reacted in a warm bath at 37°C for 15 min. After the first reaction, the tissue was dissociated by pipetting, and further reacted in a warm bath at 37°C for 5 min. After completion of the second reaction and after pipetting again, 10% KSR gfcdm medium was added, and impurities were removed by a cell strainer with a pore size of 40 µm (manufactured by Corning). The obtained cell suspension was centrifuged at 220G for 5 min, the supernatant was removed, the cells were resuspended in 10% KSR gfcdm+10 µM Y-27632, and the cells were counted. A 4 well plate (manufactured by Thermo Fisher) was coated with laminin 511E8 at 0.5 µg/cm², and the cells were seeded at a density of 5 x 10⁴ cells/cm². The serum-free medium used therefor was 10% KSR gfcdm supplemented with 10 µM Y-27632 and 10 ng/ml bFGF, and the cells were cultured under the conditions of 37°C, 5% CO₂. After culturing for 3 days, bright field observation under an inverted microscope was performed (Fig. 11C). The scale bar at the lower right of Fig. 11C shows 100 µm. As a result of observation, adhesion of the isolated cornea epithelial cells to the device and close adhesion of the cells were observed and it was shown that a corneal epithelium sheet can be formed.

### Example 11: Compound stimulability test using cell mass containing neural tissue and nonneural epithelial tissue and prepared from human ES cells

Cell masses were prepared by the method described in Example 3 from human ES cell line KhES-1. Cell masses were collected using micropipette on day 28 from the start of suspension culturing, placed in a dish for suspension culture (manufactured by SUMITOMO BAKELITE CO., LTD.), and further cultured in suspension under the conditions of 37°C, 5% CO₂ for 9 days. As the serum-free medium (gfCDM+KSR) therefor, a serum-free medium which is a 1:1 mixture of F-12+Glutamax medium (manufactured by Thermo Fisher Scientific) and IMDM+Glutamax medium (manufactured by Thermo Fisher Scientific) supplemented with 10% Knockout Serum Replacement (manufactured by Thermo Fisher Scientific), 450 µM 1-mono thioglycerol (manufactured by Wako Pure Chemical Industries, Ltd.), 1xChemically defined lipid concentrate (manufactured by Thermo Fisher Scientific), 50 unit/ml penicillin-50 µg/ml streptomycin (manufactured by Nacalai Tesque) was used.

The cell masses on day 37 of suspension culturing and prepared by the above-mentioned method were added to 1.5 ml microtubes at 3 masses per tube, and washed twice with PBS. Thereafter, a compound solution obtained by diluting a test compound with PBS to a concentration of 2.5% was added to the microtube, and the cell masses were treated with a compound for 24 hr under the conditions of 37°C, 5% CO₂. As the test compounds at this time, promethazine hydrochloride (manufactured by Sigma Aldrich) with GHS eye stimulability classified into Category 1: severe eye damage (irreversible action), and 4-formylbenzoic acid (manufactured by Tokyo Chemical Industry Co., Ltd.) with that classified into Category 2: irritating (reversible action) were used, and cell mass treated with PBS alone was used as a control without addition. The cell masses after the compound treatment were washed 3 times with PBS, further stained with a 0.02% fluorescein/PBS solution (manufactured by Sigma Aldrich) for 30 sec, and then washed 4 times with PBS. The cell masses after washing were treated with 200 µl PBS for 10 min and fluorescein incorporated in the cell masses was extracted. The concentration of fluorescein in the extract prepared by the above-mentioned method was measured using a fluorescent plate reader (2104 EnVision multilabel counter, manufactured by Perkin Elmer) under the conditions of excitation 485 nm and emission 535 nm.

As a result, a significant difference was found in the elution amount after incorporation of fluorescein between the cell masses treated with promethazine hydrochloride in Category 1 and the cell masses treated with 4-formylbenzoic acid in Category 2 (Fig. 12A). At this time, the elution amount from the cell masses treated only with PBS as a control was below the detection limit. From the above-mentioned results, it was shown that a compound stimulability test in vitro can be performed using a cell mass containing neural tissue and nonneural epithelial tissue and produced by the production method described in the present specification.

### [Industrial Applicability]

According to the present invention, a cell mass containing neural tissue such as retina and the like, neural cells and nonneural epithelial tissue such as cornea and the like can be produced efficiently from pluripotent stem cells at a low cost.

This application is based on a patent application No. 2017-226308 filed in Japan (filing date: November 24, 2017), the contents of which are incorporated in full herein.

## Claims

1. A method for producing a cell mass comprising 1) neural cells or neural tissue and 2) nonneural epithelial tissue, comprising the following steps (1) and (2):
(1) a first step of suspension-culturing pluripotent stem cells to form a cell aggregate in the presence of a Wnt signal transduction pathway inhibiting substance,
(2) a second step of suspension-culturing the aggregate obtained in the first step in the presence of a BMP signal transduction pathway activating substance, thereby obtaining a cell mass comprising 1) neural cells or neural tissue and 2) nonneural epithelial tissue.

2. A method for producing a cell mass comprising 1) neural cells or neural tissue and 2) nonneural epithelial tissue, comprising the following steps (a), (1) and (2):
(a) step a of maintenance-culturing pluripotent stem cells in the absence of feeder cells and in a medium containing 1) a TGFβ family signal transduction pathway inhibiting substance and/or a Sonic hedgehog signal transduction pathway activating substance, and 2) a factor for maintaining an undifferentiated state,
(1) a first step of suspension-culturing the pluripotent stem cells, which were maintenance-cultured in step a, to form a cell aggregate in the presence of a Wnt signal transduction pathway inhibiting substance,
(2) a second step of suspension-culturing the aggregate obtained in the first step in the presence of a BMP signal transduction pathway activating substance, thereby obtaining a cell mass comprising 1) neural cells or neural tissue and 2) nonneural epithelial tissue.

3. The production method according to claim 1 or 2, wherein the Wnt signal transduction pathway inhibiting substance is a PORCN inhibitor.

4. The production method according to any one of claims 1 to 3, wherein the culturing in the step (1) and/or the step (2) is performed in the further presence of a TGFβ signal transduction pathway inhibiting substance.

5. A cell mass comprising 1) neural cells or neural tissue and 2) nonneural epithelial tissue obtained by the production method according to any one of claims 1 to 4.

6. A method for producing a nonneural epithelial tissue sheet, comprising the following steps (1) - (4):
(1) a first step of suspension-culturing pluripotent stem cells to form a cell aggregate in the presence of a Wnt signal transduction pathway inhibiting substance,
(2) a second step of suspension-culturing the aggregate obtained in the first step in the presence of a BMP signal transduction pathway activating substance, thereby obtaining a cell mass comprising 1) neural cells or neural tissue and 2) nonneural epithelial tissue,
(3) a third step of collecting 2) nonneural epithelial tissue from the cell mass obtained in the second step,
(4) a fourth step of dispersing the 2) nonneural epithelial tissue obtained in the third step and culturing same on a flat plane, thereby obtaining a nonneural epithelial tissue sheet.

7. A method for producing a nonneural epithelial tissue sheet, comprising the following step (a) and the following steps (1) - (4) :
(a) step a of maintenance-culturing pluripotent stem cells in the absence of feeder cells and in a medium containing 1) a TGFβ family signal transduction pathway inhibiting substance and/or a Sonic hedgehog signal transduction pathway activating substance, and 2) a factor for maintaining an undifferentiated state,
(1) a first step of suspension-culturing the pluripotent stem cells, which were maintenance-cultured in step a, to form a cell aggregate in the presence of a Wnt signal transduction pathway inhibiting substance,
(2) a second step of suspension-culturing the aggregate obtained in the first step in the presence of a BMP signal transduction pathway activating substance, thereby obtaining a cell mass comprising 1) neural cells or neural tissue and 2) nonneural epithelial tissue,
(3) a third step of collecting 2) nonneural epithelial tissue from the cell mass obtained in the second step,
(4) a fourth step of dispersing the 2) nonneural epithelial tissue obtained in the third step and culturing same on a flat plane, thereby obtaining a nonneural epithelial tissue sheet.

8. The production method according to claim 6 or 7, wherein the 2) nonneural epithelial tissue is cornea or a precursor tissue thereof.

9. A nonneural epithelial tissue sheet obtained by the production method according to any one of claims 6 to 8.

10. A cell mass comprising 1) neural cells or neural tissue and 2) nonneural epithelial tissue, wherein not less than 30% of the surface of the 1) neural cells or neural tissue is coated with 2) nonneural epithelial tissue, and wherein
a space having a distance between the 1) neural cells or neural tissue and the 2) nonneural epithelial tissue on the outer side of not less than 30 µm is formed in at least a part of the surface region of the 1) neural cells or neural tissue coated with the 2) nonneural epithelial tissue.

11. The cell mass according to claim 10, wherein the 2) nonneural epithelial tissue is cornea or precursor tissue thereof.

12. The cell mass according to claim 10 or 11, wherein the 1) neural cells or neural tissue are/is central nervous system cells or tissue or precursor tissue thereof.

13. The cell mass according to claim 12, wherein the central nervous system cell or tissue is retina.

14. A method for evaluating toxicity or efficacy of a test substance, comprising a step of bringing the cell mass comprising 1) neural cells or neural tissue and 2) nonneural epithelial tissue according to any one of claims 10 to 13, or a cell mass comprising 1) neural cells or neural tissue and 2) nonneural epithelial tissue obtained by the production method according to any one of claims 1 to 4 into contact with a test substance, and
a step of detecting an influence of the test substance on the cells or tissue.

15. A method for evaluating toxicity or efficacy of a test substance, comprising a step of bringing a nonneural epithelial tissue sheet obtained by the method according to any one of claims 6 to 8 into contact with a test substance, and
a step of detecting an influence of the test substance on the nonneural epithelial tissue sheet.

16. A therapeutic drug for a disease due to a disorder of a sensory organ, comprising a cell mass obtained by the method according to any one of claims 1 to 4, or a nonneural epithelial tissue sheet obtained by the method according to any one of claims 6 to 8.
